(19) **Europäisches Patentamt European Patent Office Office européen des brevets**

(11) **EP 4 186 060 B1**

(12) **EUROPEAN PATENT SPECIFICATION**

(45) Date of publication and mention of the grant of the patent:
**29.04.2026 Bulletin 2026/18**

(21) Application number: **21847227.2**

(22) Date of filing: **23.07.2021**

(51) International Patent Classification (IPC):
**G16B 20/00** *(2019.01)* **G16B 40/10** *(2019.01)*
**G16B 30/00** *(2019.01)* **G16B 50/00** *(2019.01)*
**G16H 50/00** *(2018.01)* **C12Q 1/6869** *(2018.01)*

(52) Cooperative Patent Classification (CPC):
**G16B 30/00; G16B 40/10; C12Q 1/6869**

(86) International application number:
**PCT/US2021/042992**

(87) International publication number:
**WO 2022/020728 (27.01.2022 Gazette 2022/04)**

(54) **SYSTEMS AND METHODS FOR DETECTING AND REMOVING AGGREGATES FOR CALLING CELL-ASSOCIATED BARCODES**

SYSTEME UND VERFAHREN ZUR ERKENNUNG UND ENTFERNUNG VON AGGREGATEN ZUM ABRUFEN VON ZELLASSOZIIERTEN STRICHCODES

SYSTÈMES ET PROCÉDÉS PERMETTANT DE DÉTECTER ET D'ÉLIMINER DES AGRÉGATS POUR FAIRE APPEL À DES CODES À BARRES ASSOCIÉS À DES CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.07.2020 US 202063055864 P**
**13.11.2020 US 202063113727 P**

(43) Date of publication of application:
**31.05.2023 Bulletin 2023/22**

(73) Proprietor: **10X Genomics, Inc.**
**Pleasanton, CA 94588-3260 (US)**

(72) Inventor: **DSHKHUNYAN, Narek**
**Pleasanton, CA 94588-3260 (US)**

(74) Representative: **Hamer, Thomas Daniel et al**
**Kilburn & Strode LLP**
**Lacon London**
**84 Theobalds Road**
**London WC1X 8NL (GB)**

(56) References cited:
**WO-A2-2019/236599      WO-A2-2019/236599**
**US-A1- 2018 127 744      US-A1- 2019 262 831**
**US-A1- 2019 263 912**

• **UNKNOWN UNKNOWN: "Chromium Single Cell Gene Expression with Feature Barcoding Analysis", 31 December 2018 (2018-12-31), pages 1 - 132, XP093101202, Retrieved from the Internet <URL:https://gccri.uthscsa.edu/wp-content/uploads/sites/128/2020/04/Chromium-Single-Cell-software-training.pdf> [retrieved on 20231114]**

**Description**

<u>FIELD</u>

The embodiments provided herein are generally related to systems and methods for analysis of cells and cell features. Included among embodiments provided herein are systems and methods relating to accurate detection of cell-associated barcodes.

<u>BACKGROUND</u>

Accurate detection of cell-associated barcodes such as, for example, barcodes from partitions containing one or more cells, is a primary step in the analysis of single-cell molecular datasets from barcoded partitions. Correct cell-calling remains an important challenge for the successful analysis of unbiased genome-wide single-cell molecular datasets. However, this problem becomes even more challenging when addressing single-cell datasets with protein aggregates, due to the additional complications involved with aggregates that lead to false molecular counts.

As such, there is a need for better classification of cell-associated barcodes in a wide variety of single-cell datasets. Moreover, there is a need to identify solutions that more effectively classify cell-associated barcodes with single-cell datasets.

WO2019236599 A2 for instance, provides methods for preparing a sequencing library that includes nucleic acids from a plurality of single cells. Also provided herein are compositions, such as compositions that include the nucleic acids having three index sequences.

<u>SUMMARY</u>

In accordance with various embodiments, a method is provided for detecting aggregates from a dataset, the method comprising: receiving, by one or more processors, a dataset comprising a plurality of features, each feature including an associated barcode sequence; removing, by the one or more processors, from the dataset, features having UMI counts that that do not exceed a given UMI threshold, to produce a signal dataset including those features exceeding the given UMI threshold; identifying, by the one or more processors, a suspect barcode sequence set, wherein a barcode sequence is identified as suspect if it has a total UMI count that exceeds a given barcode threshold; detecting, by the one or more processors, aggregates from the suspect barcode sequence set, the detecting comprising determining, by the one or more processors, an aggregate candidate barcode set from the suspect barcode sequence set, wherein a suspect barcode sequence qualifies as an aggregate candidate barcode if the number of detected unique features for the suspect barcode meets a detected feature threshold, wherein the detected feature threshold is a function of the number of features in the signal dataset, and identifying, by the one or more processors, aggregates from the aggregate candidate barcode set, wherein an aggregate is identified when each detected unique feature exceeds a given aggregate UMI threshold.

In accordance with various embodiments, A non-transitory computer-readable medium storing computer instructions that, when executed by a computer, cause the computer to perform a method for detecting aggregates from a dataset, the method comprising: receiving, by one or more processors, a dataset comprising a plurality of features, each feature including an associated barcode sequence; removing, by the one or more processors, from the dataset, features having UMI counts that that do not exceed a given UMI threshold; generating, by the one or more processors, a signal dataset including those features exceeding the given UMI threshold; identifying, by the one or more processors, a suspect barcode sequence set, wherein a barcode sequence is identified as suspect if it has a total UMI count that exceeds a given barcode threshold; detecting, by the one or more processors, aggregates from the suspect barcode sequence set, the detecting comprising determining, by the one or more processors, an aggregate candidate barcode set from the suspect barcode sequence set, wherein a suspect barcode sequence qualifies as an aggregate candidate barcode if the number of detected unique features for the suspect barcode meets a detected feature threshold, wherein the detected feature threshold is a function of the number of features in the signal dataset. and identifying, by the one or more processors, aggregates from the aggregate candidate barcode set, wherein an aggregate is identified when each detected unique feature exceeds a given aggregate UMI threshold.

In accordance with various embodiments, a system for detecting aggregates from a dataset comprises, a data store configured to store a dataset comprising a plurality of features, each feature including an associated barcode sequence; a computing device communicatively connected to the data store, comprising a unique molecule filtering engine configured to: remove, from the dataset, features having UMI counts that that do not exceed a given UMI threshold; generate a signal dataset including those features exceeding the given UMI threshold; identify a suspect barcode sequence set, wherein a barcode sequence is identified as suspect if it has a total UMI count that exceeds a given barcode threshold; detect aggregates from the suspect barcode sequence set, the detecting comprising: determining an aggregate candidate barcode set from the suspect barcode sequence set, wherein a suspect barcode sequence qualifies as an aggregate candidate barcode if the number of detected unique features for the suspect barcode meets a detected feature threshold, wherein the detected feature threshold is a function of the number of features in the signal dataset, and identifying aggregates from the aggregate candidate barcode set, wherein an aggregate is identified when each detected unique feature exceeds a given aggregate UMI threshold.

## BRIEF DESCRIPTION OF THE DRAWINGS

For a more complete understanding of the principles disclosed herein, and the advantages thereof, reference is now made to the following descriptions taken in conjunction with the accompanying drawings, in which:

FIG. 1 is a schematic illustration of non-limiting examples of the sequencing workflow for using single cell sequencing analysis to generate sequencing data, in accordance with various embodiments.
FIG. 2 is a non-limiting exemplary flowchart showing a process flow for conducting single cell sequencing analysis, in accordance with various embodiments.
FIG. 3a is a non-limiting exemplary flowchart showing a process flow for conducting aggregate detection and removal, in accordance with various embodiments.
FIG. 3b is a non-limiting exemplary flowchart showing a process flow for conducting aggregate detection, in accordance with various embodiments.
FIG. 4a is a non-limiting exemplary flowchart showing a process flow for conducting aggregate detection and removal, in accordance with various embodiments.
FIG. 4b is a non-limiting exemplary flowchart showing a process flow for conducting aggregate detection, in accordance with various embodiments.
FIG. 5 is a non-limiting exemplary showing a process flow for detecting aggregates from a dataset, in accordance with various embodiments.
FIG. 6 is a block diagram illustrating a computer system for use in performing methods provided herein, in accordance with various embodiments.

It is to be understood that the figures are not necessarily drawn to scale, nor are the objects in the figures necessarily drawn to scale in relationship to one another. The figures are depictions that are intended to bring clarity and understanding to various embodiments of apparatuses, systems, and methods disclosed herein. Wherever possible, the same reference numbers will be used throughout the drawings to refer to the same or like parts. Moreover, it should be appreciated that the drawings are not intended to limit the scope of the present teachings in any way.
The above-identified figures are provided by way of representation and not limitation. The figures may show simplified or partial views, and the dimensions of elements in the figures may be exaggerated or otherwise not in proportion. In addition, as the terms "on," "attached to," "connected to," "coupled to," or similar words are used herein, one element (e.g., a material, a layer, a substrate, etc.) can be "on," "attached to," "connected to," or "coupled to" another element regardless of whether the one element is directly on, attached to, connected to, or coupled to the other element or there are one or more intervening elements between the one element and the other element. In addition, where reference is made to a list of elements (e.g., elements a, b, c), such reference is intended to include any one of the listed elements by itself, any combination of less than all of the listed elements, and/or a combination of all of the listed elements. Section divisions in the specification are for ease of review only and do not limit any combination of elements discussed.

## DETAILED DESCRIPTION

The following description of various embodiments is exemplary and explanatory only and is not to be construed as limiting or restrictive in any way. Other embodiments, features, objects, and advantages of the present teachings will be apparent from the description and accompanying drawings, and from the claims.
In general, the methods and systems provided herein accomplish removing barcodes that are affected by protein aggregates from future analysis. For example, a sequence dataset obtained from a protein library such as an antibody capture library may be used herein. Protein aggregates in antibody staining agents may cause a few Gel bead-in-EMulsion ("GEM") to have extremely high molecular counts (counts of unique molecular identifiers or UMIs). Protein libraries such as antibody capture libraries can have high counts of sequence reads that may be affected by aggregates, which may cause errors in future analysis.
In addition, the method and systems provided herein can enable cost saving, especially for immunology research without the need of gene expression data. The method and systems provided herein can also allow a quick quality control test to check cell viability or cell protocol without the need to analyze a gene expression dataset at the same time with the antibody capture library sequence dataset.
Provided herein are methods and systems for removing aggregates from a sequence dataset that can be used for cell feature analysis such as a cell surface protein expression level analysis using an antibody capture library. It should be appreciated, however, that although the systems and methods disclosed herein refer to their application in antibody capture libraries for cell surface proteins, they are equally applicable to other analogous fields, like any protein-based assays such as dextramers, antigens, and intracellular proteins. Other libraries that may be used for the methods and systems provided herein may include multiplexing tags and CRISPR (clustered regularly interspaced short palindromic repeats) assays.

The disclosure, however, is not limited to these exemplary embodiments and applications or to the manner in which the exemplary embodiments and applications operate or are described herein. Moreover, the figures may show simplified or partial views, and the dimensions of elements in the figures may be exaggerated or otherwise not in proportion. In addition, as the terms "on," "attached to," "connected to," "coupled to," or similar words are used herein, one element (e.g., a material, a layer, a substrate, etc.) can be "on," "attached to," "connected to," or "coupled to" another element regardless of whether the one element is directly on, attached to, connected to, or coupled to the other element or there are one or more intervening elements between the one element and the other element. In addition, where a reference is made to a list of elements (e.g., elements a, b, c), such reference is intended to include any one of the listed elements by itself, any combination of less than all the listed elements, and/or a combination of all the listed elements. Section divisions in the specification are for ease of review only and do not limit any combination of elements discussed.

It should be understood that any use of subheadings herein is for organizational purposes and should not be read to limit the application of those sub headed features to the various embodiments herein. Each feature described herein is applicable and usable in all the various embodiments discussed herein and that all features described herein can be used in any contemplated combination, regardless of the specific example embodiments that are described herein. It should further be noted that exemplary description of specific features is used, largely for informational purposes, and not in any way to limit the design, sub feature, and functionality of the specifically described feature.

As used herein, "substantially" means sufficient to work for the intended purpose. The term "substantially" thus allows for minor, insignificant variations from an absolute or perfect state, dimension, measurement, result, or the like such as would be expected by a person of ordinary skill in the field but that do not appreciably affect overall performance. When used with respect to numerical values or parameters or characteristics that can be expressed as numerical values, "substantially" means within ten percent.

The term "ones" means more than one unless otherwise specified.

As used herein, the term "plurality" can be 2, 3, 4, 5, 6, 7, 8, 9, 10, or more.

As used herein, the terms "comprise", "comprises", "comprising", "contain", "contains", "containing", "have", "having" "include", "includes", and "including" and their variants are not intended to be limiting, are inclusive or open-ended and do not exclude additional, un-recited additives, components, integers, elements or method steps. For example, a process, method, system, composition, kit, or apparatus that comprises a list of features is not necessarily limited only to those features but may include other features not expressly listed or inherent to such process, method, system, composition, kit, or apparatus.

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

Unless otherwise defined, scientific and technical terms used in connection with the present teachings described herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well-known and commonly used in the art. Standard techniques are used, for example, for nucleic acid purification and preparation, chemical analysis, recombinant nucleic acid, and oligonucleotide synthesis. Enzymatic reactions and purification techniques are performed according to manufacturer's specifications or as commonly accomplished in the art or as described herein. Standard molecular biological techniques and procedures described herein are generally performed according to conventional methods well known in the art and as described in various general and more specific references that are cited and discussed throughout the instant specification. See, e.g., Sambrook et al., Molecular Cloning: A Laboratory Manual (Third ed., Cold Spring Harbor Laboratory Press, Cold Spring Harbor, N.Y. 2000). The nomenclatures utilized in connection with, and the laboratory procedures and standard techniques described herein are those well-known and commonly used in the art.

As used herein, the term "unique molecular identifier" or "UMI" refers to a molecular tag (e.g., a nucleotide sequence) that is attached to a unique DNA or RNA fragment or antibody prior to PCR amplification. After sequencing, they are used to distinguish sequenced reads from unique DNA or RNA fragments or antibody versus PCR duplicates.

An "antibody capture library" refers to a library prepared using a plurality of antibody-oligonucleotide conjugates and compatible with a single cell RNA sequencing platform. For example, each of the conjugates comprises an antibody conjugated to a nucleotide sequence that comprises a barcode sequence, a capture sequence, a unique molecular identifier sequence, or a combination thereof.

The term "molecular count" or "UMI count" refers to a total number of unique molecules identified, such as unique DNA or RNA fragments or antibody molecules.

A "polynucleotide", "nucleic acid", or "oligonucleotide" refers to a linear polymer of nucleosides (including deoxyribonucleosides, ribonucleotides, or analogs thereof) joined by internucleonic linkages. Typically, a polynucleotide comprises at least three nucleosides. Usually oligonucleotides range in size from a few monomeric units, e.g. 3-4, to several hundreds of monomeric units. Whenever a polynucleotide such as an oligonucleotide is represented by a sequence of letters, such as "ATGCCTG," it will be understood that the nucleotides are in 5'->3' order from left to right and that "A" denotes deoxyadenosine, "C" denotes deoxycytidine, "G" denotes deoxy guanosine, and "T" denotes thymidine, unless otherwise noted. The letters A, C, G, and T may be used to refer to the bases themselves, to nucleosides, or to nucleotides comprising the bases, as is standard in the art.

DNA (deoxyribonucleic acid) is a chain of nucleotides containing 4 types of nucleotides; A (adenine), T (thymine), C (cytosine), and G (guanine), and RNA (ribonucleic acid) is comprised of 4 types of nucleotides; A, U (uracil), G, and C. Certain pairs of nucleotides specifically bind to one another in a complementary fashion (called complementary base pairing). That is, adenine (A) pairs with thymine (T) (in the case of RNA, however, adenine (A) pairs with uracil (U)), and cytosine (C) pairs with guanine (G). When a first nucleic acid strand binds to a second nucleic acid strand made up of nucleotides that are complementary to those in the first strand, the two strands bind to form a double strand. As used herein, "nucleic acid sequencing data," "nucleic acid sequencing information," "nucleic acid sequence," "genomic sequence," "genetic sequence," or "fragment sequence," or "nucleic acid sequencing read" denotes any information or data that is indicative of the order of the nucleotide bases (e.g., adenine, guanine, cytosine, and thymine/uracil) in a molecule (e.g., whole genome, whole transcriptome, exome, oligonucleotide, polynucleotide, fragment, etc.) of DNA or RNA. The present teachings contemplate sequence information obtained using all available varieties of techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, electronic signature-based systems, etc.

As used herein, the term "cell" is used interchangeably with the term "biological cell." Non-limiting examples of biological cells include eukaryotic cells, plant cells, animal cells, such as mammalian cells, reptilian cells, avian cells, fish cells or the like, prokaryotic cells, bacterial cells, fungal cells, protozoan cells, or the like, cells dissociated from a tissue, such as muscle, cartilage, fat, skin, liver, lung, neural tissue, and the like, immunological cells, such as T cells, B cells, natural killer cells, macrophages, and the like, embryos (e.g., zygotes), oocytes, ova, sperm cells, hybridomas, cultured cells, cells from a cell line, cancer cells, infected cells, transfected and/or transformed cells, reporter cells and the like. A mammalian cell can be, for example, from a human, mouse, rat, horse, goat, sheep, cow, primate or the like.

A term "genome', as used herein, refers to the genetic material of a cell or organism, including animals, such as mammals, e.g., humans and comprises nucleic acids, such as DNA. In humans, total DNA includes, for example, genes, noncoding DNA and mitochondrial DNA. The human genome typically contains 23 pairs of linear chromosomes: 22 pairs of autosomal chromosomes (autosomes) plus the sex-determining X and Y chromosomes. The 23 pairs of chromosomes include one copy from each parent. The DNA that makes up the chromosomes is referred to as chromosomal DNA and is present in the nucleus of human cells (nuclear DNA). Mitochondrial DNA is in mitochondria as a circular chromosome, is inherited from only the female parent, and is often referred to as the mitochondrial genome as compared to the nuclear genome of DNA located in the nucleus.

As used herein, "cell features" include cell surface features or intracellular features, such as peptides, proteins, genes or nucleic acids such as RNA or DNA. Cell surface features may include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherent junction, or any combination thereof. In some instances, cell features may include intracellular analytes, such as proteins, protein modifications (e.g., phosphorylation status or other post-translational modifications), nuclear proteins, nuclear membrane proteins, or any combination thereof.

As used herein, "a labelling agent" may include any molecule that can bind to a cell and label a cell or a cell feature. A labelling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell surface feature to which the binding group binds. For example, the reporter oligonucleotide may comprise a barcode sequence that permits identification of the labelling agent. For example, a labelling agent that is specific to one type of cell feature (e.g., a first cell surface feature) may have coupled thereto a first reporter oligonucleotide, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 20190177800; and U.S. Pat. Pub. 20190367969.

The phrase "next generation sequencing" (NGS) refers to sequencing technologies having increased throughput as compared to traditional Sanger- and capillary electrophoresis-based approaches, for example with the ability to generate hundreds of thousands of relatively small sequence reads at a time. Some examples of next generation sequencing techniques include, but are not limited to, sequencing by synthesis, sequencing by ligation, and sequencing by hybridization. More specifically, the MISEQ, HISEQ and NEXTSEQ Systems of Illumina and the Personal Genome Machine (PGM), Ion Torrent, and SOLiD Sequencing System of Life Technologies Corp, provide massively parallel sequencing of whole or targeted genomes. The SOLiD System and associated workflows, protocols, chemistries, etc. are described in more detail in PCT Publication No. WO 2006/084132, entitled "Reagents, Methods, and Libraries for Bead-Based Sequencing," international filing date Feb. 1, 2006, U.S. patent application Ser. No. 12/873,190, entitled "Low-Volume Sequencing System and Method of Use," filed on Aug. 31, 2010, and U.S. patent application Ser. No. 12/873,132, entitled "Fast-Indexing Filter Wheel and Method of Use," filed on Aug. 31, 2010.

The phrase "sequencing run" refers to any step or portion of a sequencing process performed to determine some information relating to at least one biomolecule (e.g., nucleic acid molecule). The term can also refer to a flow cell containing data from one sequencing instrument run. The sequencing data can be further addressed by lane and by one or more sample indices.

The term "read" or "sequencing read" with reference to nucleic acid sequencing refers to the sequence of nucleotides determined for a nucleic acid fragment that has been subjected to sequencing, such as, for example, next generation sequencing ("NGS"). Reads can be any a sequence of any number of nucleotides which defines the read length.

Methods of processing and sequencing nucleic acids in accordance with the methods and systems described in the present application are also described in further detail in U.S. Ser. Nos. 14/316,383; 14/316,398; 14/316,416; 14/316,431; 14/316,447; and 14/316,463.

The term "barcode," as used herein, generally refers to a label, or identifier, that conveys or is capable of conveying information about an analyte. A barcode can be part of an analyte. A barcode can be independent of an analyte. A barcode can be a tag attached to an analyte (e.g., nucleic acid molecule) or a combination of the tag in addition to an endogenous characteristic of the analyte (e.g., size of the analyte or end sequence(s)). A barcode may be unique. Barcodes can have a variety of different formats. For example, barcodes can include barcode sequences, such as: polynucleotide barcodes; random nucleic acid and/or amino acid sequences; and synthetic nucleic acid and/or amino acid sequences. A barcode can be attached to an analyte in a reversible or irreversible manner. A barcode can be added to, for example, a fragment of a deoxyribonucleic acid (DNA) or ribonucleic acid (RNA) sample before, during, and/or after sequencing of the sample. Barcodes can allow for identification and/or quantification of individual sequencing reads.

As used herein, the term "cell barcode" refers to any barcodes that have been determined to be associated with a cell, as determined by a "cell calling" step within various embodiments of the disclosure.

As used herein, the term "Gel bead-in-EMulsion" or "GEM" refers to a droplet containing some sample volume and a barcoded gel bead, forming an isolated reaction volume. When referring to the subset of the sample contained in the droplet, the term "partition" may also be used. In various embodiments within the disclosure, the term barcode can refer to a GEM containing a gel bead that carries many DNA oligonucleotides with the same barcode, whereas different GEMs have different barcodes.

As used herein, the term "GEM well" or "GEM group" refers to a set of partitioned cells (i.e., Gel beads-in-Emulsion or GEMs) from a single 10x Chromium™ Chip channel. One or more sequencing libraries can be derived from a GEM well.

The terms "adaptor(s)", "adapter(s)" and "tag(s)" may be used synonymously. An adaptor or tag can be coupled to a polynucleotide sequence to be "tagged" by any approach, including ligation, hybridization, or other approaches. In various embodiments within the disclosure, the term adapter can refer to customized strands of nucleic acid base pairs created to bind with specific nucleic acid sequences, e.g., sequences of DNA.

The term "bead," as used herein, generally refers to a particle. The bead may be a solid or semi-solid particle. The bead may be a gel bead. The gel bead may include a polymer matrix (e.g., matrix formed by polymerization or cross-linking). The polymer matrix may include one or more polymers (e.g., polymers having different functional groups or repeat units). Polymers in the polymer matrix may be randomly arranged, such as in random copolymers, and/or have ordered structures, such as in block copolymers. Cross-linking can be via covalent, ionic, or inductive, interactions, or physical entanglement. The bead may be a macromolecule. The bead may be formed of nucleic acid molecules bound together. The bead may be formed via covalent or non-covalent assembly of molecules (e.g., macromolecules), such as monomers or polymers. Such polymers or monomers may be natural or synthetic. Such polymers or monomers may be or include, for example, nucleic acid molecules (e.g., DNA or RNA). The bead may be formed of a polymeric material. The bead may be magnetic or nonmagnetic. The bead may be rigid. The bead may be flexible and/or compressible. The bead may be disruptable or dissolvable. The bead may be a solid particle (e.g., a metal-based particle including but not limited to iron oxide, gold or silver) covered with a coating comprising one or more polymers. Such coating may be disruptable or dissolvable.

The term "macromolecule" or "macromolecular constituent," as used herein, generally refers to a macromolecule contained within or from a biological particle. The macromolecular constituent may comprise a nucleic acid. In some cases, the biological particle may be a macromolecule. The macromolecular constituent may comprise DNA. The macromolecular constituent may comprise RNA. The RNA may be coding or non-coding. The RNA may be messenger RNA (mRNA), ribosomal RNA (rRNA) or transfer RNA (tRNA), for example. The RNA may be a transcript. The RNA may be small RNA that are less than 200 nucleic acid bases in length, or large RNA that are greater than 200 nucleic acid bases in length. Small RNAs may include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA) and small rDNA-derived RNA (srRNA). The RNA may be double-stranded RNA or single-stranded RNA. The RNA may be circular RNA. The macromolecular constituent may comprise a protein. The macromolecular constituent may comprise a peptide. The macromolecular constituent may comprise a polypeptide.

The term "molecular tag," as used herein, generally refers to a molecule capable of binding to a macromolecular constituent. The molecular tag may bind to the macromolecular constituent with high affinity. The molecular tag may bind to the macromolecular constituent with high specificity. The molecular tag may comprise a nucleotide sequence. The molecular tag may comprise a nucleic acid sequence. The nucleic acid sequence may be at least a portion or an entirety of the molecular tag. The molecular tag may be a nucleic acid molecule or may be part of a nucleic acid molecule. The molecular tag may be an oligonucleotide or a polypeptide. The molecular tag may comprise a DNA aptamer. The molecular tag may be or comprise a primer. The molecular tag may be, or comprise, a protein. The molecular tag may comprise a polypeptide. The molecular tag may be a barcode.

The term "partition," as used herein, generally, refers to a space or volume that may be suitable to contain one or more species or conduct one or more reactions. A partition may be a physical compartment, such as a droplet or well. The partition may isolate space or volume from another space or volume. The droplet may be a first phase (e.g., aqueous phase) in a second phase (e.g., oil) immiscible with the first phase. The droplet may be a first phase in a second phase that does not phase separate from the first phase, such as, for example, a capsule or liposome in an aqueous phase. A partition may comprise one or more other (inner) partitions. In some cases, a partition may be a virtual compartment that can be defined and identified by an index (e.g., indexed libraries) across multiple and/or remote physical compartments. For example, a physical compartment may comprise a plurality of virtual compartments.

The term "subject," as used herein, generally refers to an animal, such as a mammal (e.g., human) or avian (e.g., bird), or other organism, such as a plant. For example, the subject can be a vertebrate, a mammal, a rodent (e.g., a mouse), a primate, a simian or a human. Animals may include, but are not limited to, farm animals, sport animals, and pets. A subject can be a healthy or asymptomatic individual, an individual that has or is suspected of having a disease (e.g., cancer) or a pre-disposition to the disease, and/or an individual that is in need of therapy or suspected of needing therapy. A subject can be a patient. A subject can be a microorganism or microbe (e.g., bacteria, fungi, archaea, viruses).

The term "sample," as used herein, generally refers to a "biological sample" of a subject. The sample may be obtained from a tissue of a subject. The sample may be a cell sample. A cell may be a live cell. The sample may be a cell line or cell culture sample. The sample can include one or more cells. The sample can include one or more microbes. The biological sample may be a nucleic acid sample or protein sample. The biological sample may also be a carbohydrate sample or a lipid sample. The biological sample may be derived from another sample. The sample may be a tissue sample, such as a biopsy, core biopsy, needle aspirate, or fine needle aspirate. The sample may be a fluid sample, such as a blood sample, urine sample, or saliva sample. The sample may be a skin sample. The sample may be a cheek swab. The sample may be a plasma or serum sample. The sample may be a cell-free or cell free sample. A cell-free sample may include extracellular polynucleotides. Extracellular polynucleotides may be isolated from a bodily sample that may be selected from the group consisting of blood, plasma, serum, urine, saliva, mucosal excretions, sputum, stool and tears. In some embodiments, the term "sample" can refer to a cell or nuclei suspension extracted from a single biological source (blood, tissue, etc.).

The sample may comprise any number of macromolecules, for example, cellular macromolecules. The sample may include one or more constituents of a cell and may not include other constituents of the cell. An example of such cellular constituents is a nucleus or an organelle. The sample may be or may include DNA, RNA, organelles, proteins, or any combination thereof. The sample may be or include a chromosome or other portion of a genome. The sample may be or may include a bead (e.g., a gel bead) comprising a cell or one or more constituents from a cell, such as DNA, RNA, nucleus, organelles, proteins, or any combination thereof, from the cell. The sample may be or may include a matrix (e.g., a gel or polymer matrix) comprising a cell or one or more constituents from a cell, such as DNA, RNA, a cell nucleus, organelles, proteins, or any combination thereof, from the cell.

As used herein, the term "PCR duplicates" refers to duplicates created during PCR amplification. During PCR amplification of the fragments, each unique fragment that is created may result in multiple read-pairs sequenced with near identical barcodes and sequence data. These duplicate reads are identified computationally and are collapsed into a single fragment record for downstream analysis.

## SINGLE CELL SEQUENCING AND DATA ANALYSIS WORKFLOWS

### *Single Cell Sequencing Workflow*

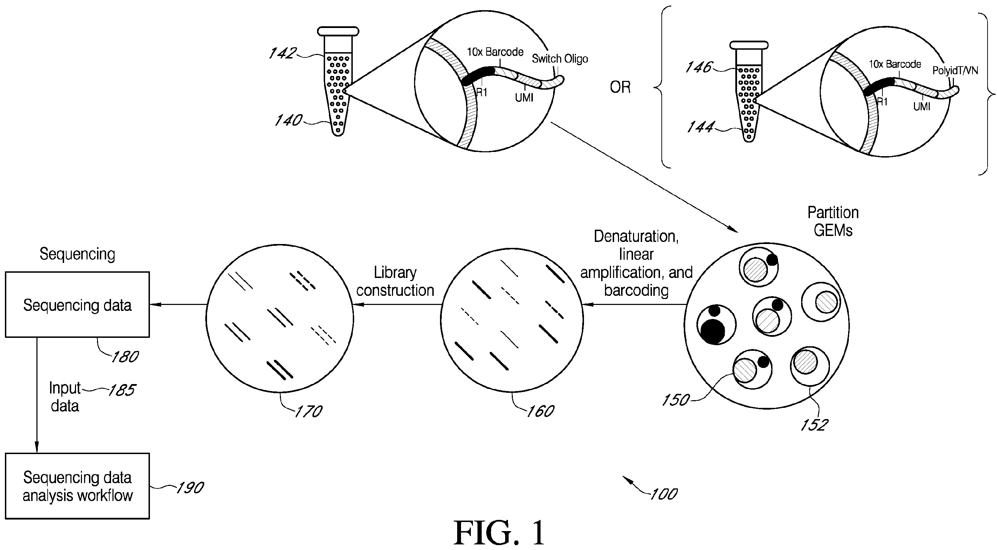

In accordance with various embodiments, a general schematic workflow is provided in FIG. 1 to illustrate a non-limiting example process for using single cell sequencing technology to generate sequencing data. Such sequencing data can be used for charactering cells and cell features in accordance with various embodiments. The workflow can include various combinations of features, whether it has more or less features than that illustrated in FIG. 1. As such, FIG. 1 simply illustrates one example of a possible workflow.

### *GEM Generation*

The workflow 100 provided in FIG. 1 begins with Gel beads-in-EMulsion (GEMs) generation. The bulk cell suspension containing the cells is mixed with a gel beads solution 140 or 144 containing a plurality of individually barcoded gel beads 142 or 146. In various embodiments, this step results in partitioning the cells into a plurality of individual GEMs 150, each including a single cell, and a barcoded gel bead 142 or 146. This step also results in a plurality of GEMs 152, each containing a barcoded gel bead 142 or 146 but no nuclei. Detail related to GEM generation, in accordance with various embodiments disclosed herein, is provided below. Further details can be found in US Patent Nos. 10343166 and 10583440, US Published Application Nos. US20180179590A1, US20190367969A1, US20200002763A1, and US20200002764A1, and Published International PCT Application No. WO 2019/040637,.

In various embodiments, GEMs can be generated by combining barcoded gel beads, individual cells, and other reagents or a combination of biochemical reagents that may be necessary for the GEM generation process. Such reagents may include, but are not limited to, a combination of biochemical reagents (e.g., a master mix) suitable for GEM generation and partitioning oil. The barcoded gel beads 142 or 146 of the various embodiments herein may include a gel bead attached to oligonucleotides containing (i) an Illumina® P5 sequence (adapter sequence), (ii) a 16 nucleotide (nt) 10x Barcode, and (iii) a Read 1 (Read 1N) sequencing primer sequence. It is understood that other adapter, barcode, and sequencing primer sequences can be contemplated within the various embodiments herein.

In various embodiments, GEMS are generated by partitioning the cells using a microfluidic chip. To achieve single cell resolution per GEM, the cells can be delivered at a limiting dilution, such that the majority (e.g., ~90-99%) of the generated GEMs do not contain any cells, while the remainder of the generated GEMs largely contain a single cell.

In the methods and systems described herein, one or more labelling agents capable of binding to or otherwise coupling to one or more cell features may be used to characterize cells and/or cell features in combination with GEMs 152. In various embodiments, the one or more labelling agents may include barcoded nucleic acid molecules, or derivatives generated therefrom, which can then be sequenced on a suitable sequencing platform to obtain datasets of sequence reads for future analysis described herein.

In various embodiments, a library of potential cell feature labelling agents may be provided associated with nucleic acid reporter molecules, e.g., where a different reporter oligonucleotide sequence is associated with each labelling agent capable of binding to a specific cell feature. The cell feature labelling agents may comprise a functional sequence that can be configured to hybridize to a commentary sequence present on a nucleotide acid barcode molecule on individually barcoded gel beads 142 or 146.

In some aspects, different members of the library may be characterized by the presence of a different oligonucleotide sequence label, e.g., an antibody capable of binding to a first type of protein may have associated with it a first known reporter oligonucleotide sequence, while an antibody capable of binding to a second protein (i.e., different than the first protein) may have a different known reporter oligonucleotide sequence associated with it.

Prior to partitioning, the cells may be incubated with the library of labelling agents, that may represent labelling agents to a broad panel of different cell features, e.g., receptors, proteins, etc., and which include their associated reporter oligonucleotides. Unbound labelling agents may be washed from the cells, and the cells may then be co-partitioned (e.g., into droplets or wells) along with partition-specific barcode oligonucleotides (e.g., attached to a bead, such as a gel bead). As a result, the partitions may include the cell or cells, as well as the bound labelling agents and their known, associated reporter oligonucleotides.

In other instances, e.g., to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide. In this way, different samples or groups can be independently processed and subsequently combined for pooled analysis (e.g., partition-based barcoding as described elsewhere herein). See, e.g., U.S. Pat. Pub. 20190323088.

***Barcoding RNA Molecules of Fragments***

The workflow 100 provided in FIG. 1A further includes lysing the cells and barcoding the RNA molecules or fragments for producing a plurality of uniquely barcoded single-stranded nucleic acid molecules or fragments. Upon generation of the GEMs 150, the gel beads 142 or 146 can be dissolved releasing the various oligonucleotides of the embodiments described above, which are then mixed with the RNA molecules or fragments resulting in a plurality of uniquely barcoded single-stranded nucleic acid molecules or fragments 160 following a nucleic acid extension reaction, e.g., reverse transcription of mRNA to cDNA, within the GEMs 150. Detail related to generation of the plurality of uniquely barcoded single-stranded nucleic acid molecules or fragments 160, in accordance with various embodiments disclosed herein, is provided below.

In various embodiments, upon generation of the GEMs 150, the gel beads 142 or 146 can be dissolved, and oligonucleotides of the various embodiments disclosed herein, containing a capture sequence, e.g., a poly(dT) sequence or a template switch oligonucleotide (TSO) sequence, a unique molecular identifier (UMI), a unique 10x Barcode, and a Read 1 sequencing primer sequence can be released and mixed with the RNA molecules or fragments and other reagents or a combination of biochemical reagents (e.g., a master mix necessary for the nucleic acid extension process). Denaturation and a nucleic acid extension reaction, e.g., reverse transcription, within the GEMs can then be performed to produce a plurality of uniquely barcoded single-stranded nucleic acid molecules or fragments 160. In various embodiments herein, the plurality of uniquely barcoded single-stranded nucleic acid molecules or fragments 160 can be 10x barcoded single-stranded nucleic acid molecules or fragments. In one non-limiting example of the various embodiments herein, a pool of ~750,000, 10x barcodes are utilized to uniquely index and barcode nucleic acid molecules derived from the RNA molecules or fragments of each individual cell.

Accordingly, the in-GEM barcoded nucleic acid products of the various embodiments herein can include a plurality of 10x barcoded single-stranded nucleic acid molecules or fragments that can be subsequently removed from the GEM environment and amplified for library construction, including the addition of adaptor sequences for downstream sequencing. In one non-limiting example of the various embodiments herein, each such in-GEM 10x barcoded single-stranded nucleic acid molecule or fragment can include a unique molecular identifier (UMI), a unique 10x barcode, a Read 1 sequencing primer sequence, and a fragment or insert derived from an RNA fragment of the cell, e.g., cDNA from an mRNA via reverse transcription. Additional adaptor sequence may be subsequently added to the in-GEM barcoded nucleic acid molecules after the GEMs are broken.

In various embodiments, after the in-GEM barcoding process, the GEMs 150 are broken and pooled barcoded nucleic acid molecules or fragments are recovered. The 10x barcoded nucleic acid molecules or fragments are released from the droplets, i.e., the GEMs 150, and processed in bulk to complete library preparation for sequencing, as described in detail below. In various embodiments, following the amplification process, leftover biochemical reagents can be removed from the post-GEM reaction mixture. In one embodiment of the disclosure, silane magnetic beads can be used to remove leftover biochemical reagents. Additionally, in accordance with embodiments herein, the unused barcodes from the sample can be eliminated, for example, by Solid Phase Reversible Immobilization (SPRI) beads.

***Library Construction***

The workflow 100 provided in FIG. 1A further includes a library construction step. In the library construction step of workflow 100, a library 170 containing a plurality of double-stranded DNA molecules or fragments are generated. These double-stranded DNA molecules or fragments can be utilized for completing the subsequent sequencing step. Detail related to the library construction, in accordance with various embodiments disclosed herein, is provided below.

In accordance with various embodiments disclosed herein, an Illumina® P7 sequence and P5 sequence (adapter sequences), a Read 2 (Read 2N) sequencing primer sequence, and a sample index (SI) sequence(s) (e.g., i7 and/or i5) can be added during the library construction step via PCR to generate the library 170, which contains a plurality of double stranded DNA fragments. In accordance with various embodiments herein, the sample index sequences can each comprise of one or more oligonucleotides. In one embodiment, the sample index sequences can each comprise of four to eight or more oligonucleotides. In various embodiments, when analyzing the single cell sequencing data for a given sample, the reads associated with all four of the oligonucleotides in the sample index can be combined for identification of a sample. Accordingly, in one non-limiting example, the final single cell gene expression analysis sequencing libraries contain sequencer compatible double-stranded DNA fragments containing the P5 and P7 sequences used in Illumina® bridge amplification, sample index (SI) sequence(s) (e.g., i7 and/or i5), a unique 10x barcode sequence, and Read 1 and Read 2 sequencing primer sequences.

Various embodiments of single cell sequencing technology within the disclosure can at least include platforms such as One Sample, One GEM Well, One Flowcell; One Sample, One GEM well, Multiple Flowcells; One Sample, Multiple GEM Wells, One Flowcell; Multiple Samples, Multiple GEM Wells, One Flowcell; and Multiple Samples, Multiple GEM Wells, Multiple Flowcells platform. Accordingly, various embodiments within the disclosure can include sequence dataset from one or more samples, samples from one or more donors, and multiple libraries from one or more donors.

The workflow 100 provided in FIG. 1 further includes a sequencing step. In this step, the library 170 can be sequenced to generate a plurality of sequencing data 180. The fully constructed library 170 can be sequenced according to a suitable sequencing technology, such as a next-generation sequencing protocol, to generate the sequencing data 180. In various embodiments, the next-generation sequencing protocol utilizes the Ilumina® sequencer for generating the sequencing data. It is understood that other next-generation sequencing protocols, platforms, and sequencers such as, e.g., MiSeq™, NextSeq™ 500/550 (High Output), HiSeq 2500™ (Rapid Run), HiSeq™ 3000/4000, and NovaSeq™, can be also used with various embodiments herein.

### Sequencing Data Input and Data Analysis Workflow

The workflow 100 provided in FIG. 1 further includes a sequencing data analysis workflow 190. With the sequencing data 180 in hand, the data can then be output, as desired, and used as an input data 185 for the downstream sequencing data analysis workflow 190, in accordance with various embodiments herein. Sequencing the single cell libraries produces standard output sequences (also referred to as the "sequencing data", "sequence data", or the "sequence output data") that can then be used as the input data 185, in accordance with various embodiments herein. The sequence data contains sequenced fragments (also interchangeably referred to as "fragment sequence reads", "sequencing reads" or "reads"), which in various embodiments include RNA sequences of the RNA fragments containing the associated 10x barcode sequences, adapter sequences, and primer oligo sequences.

The various embodiments, systems and methods within the disclosure further include processing and inputting the sequence data. A compatible format of the sequencing data of the various embodiments herein can be a FASTQ file. Other file formats for inputting the sequence data is also contemplated within the disclosure herein. Various software tools within the embodiments herein can be employed for processing and inputting the sequencing output data into input files for the downstream data analysis workflow. It is understood that, various systems and methods with the embodiments herein are contemplated that can be employed to independently analyze the inputted single cell sequencing data for studying cells and cell features in accordance with various embodiments.

## CELL FEATURE ANALYSIS WORKFLOW

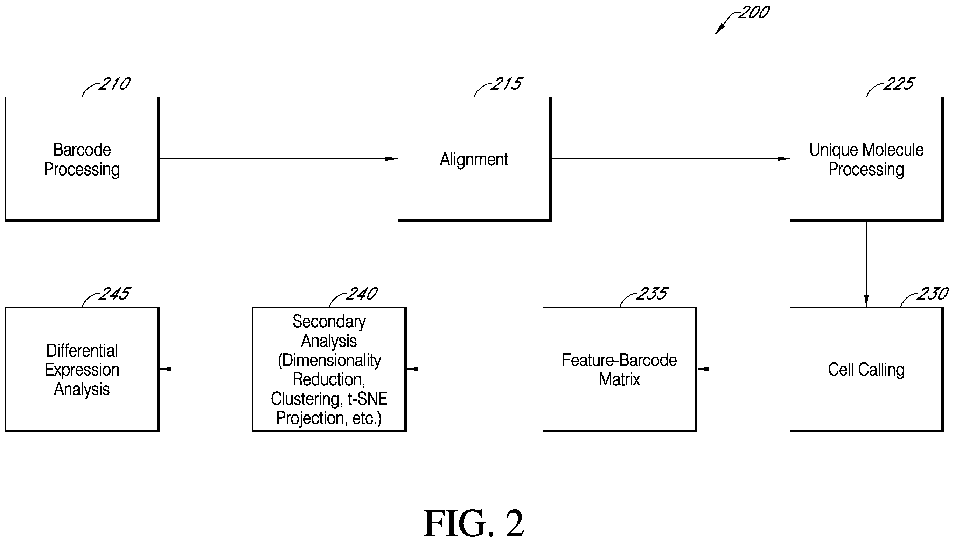

In accordance with various embodiments, a general schematic workflow is provided in FIG. 2 to illustrate a non-limiting example process of a sequencing data analysis workflow for cell feature analysis. The workflow can include various combinations of features, whether it be more or less features than that illustrated in FIG. 2. As such, FIG. 2 simply illustrates one example of a possible workflow for conducting cell feature analysis.

FIG. 2 provides a schematic workflow 200 for conducting cell feature analysis. It should be appreciated that the methodologies described in the workflow 200 of FIG. 2 and accompanying descriptions can be implemented independently of the methodologies for generating single cell sequencing data described in general. Therefore, FIG. 2 can be implemented independently of a sequencing data generating workflow if it is capable of sufficiently analyzing single cell sequencing data sets for cell feature analysis.

Moreover, the data analysis workflow can include one or more of the analysis steps illustrated in FIG. 2. Not all the steps within the disclosure of FIG. 2 need to be utilized as a group. Therefore, some of the steps within FIG. 2 are capable of independently performing the necessary data analysis as part of the various embodiments disclosed herein. Accordingly, it is understood that, certain steps within the disclosure can be used either independently or in combination with other steps within the disclosure, while certain other steps within the disclosure can only be used in combination with certain other steps within the disclosure. Further, one or more of the steps or filters described below, presumably defaulted to be utilized as part of the computational pipeline, can also not be utilized per user input. It is understood that the reverse is also contemplated. It is further understood that additional steps for analyzing the generated sequencing data are also contemplated as part of the computational pipeline within the disclosure.

### Barcode Processing

The workflow 200 can comprise, at step 210, processing the barcodes in the single cell sequencing data set for fixing the occasional sequencing error in the barcodes so that the sequenced fragments can be associated with the original barcodes, thus improving the data quality. Detail related to the barcode processing and correction as part of the various embodiments disclosed herein is provided below.

In accordance with various embodiments, the barcode sequence can be between about 2bp to about 25bp. In accordance with various other embodiments, the barcode sequence can be between about 5bp and 20bp. In accordance with various preferred embodiments, the barcode sequence can be between about 10bp and 16bp. The length of the barcode sequence can affect the number of unique barcodes present in the sequencing library. Accordingly, it is understood that barcode sequences shorter than 10bp can be selected in accordance with various embodiments herein, provided that the read sequence data from multiple cells are not associated with the same barcode because of severe lack of diversity caused by a shorter length of the barcode sequence. The barcode sequence can be obtained from the "I2" index read and is read as part of the I2 reaction. Accordingly, it is understood that barcode sequences longer than 16bp can be selected in accordance with various embodiments herein, provided that the barcode sequence length is within the limits of the I2 index read and reaction, and that it can be sequenced on a sequencer within the various embodiments herein. The barcode processing step can include checking each barcode sequence against a "whitelist" of correct barcode sequences. The barcode processing step can further include counting the frequency of each whitelist barcode. The barcode processing step can also include various barcode correction steps as part of the various embodiments disclosed herein. For example, one may attempt to correct the barcodes that are not included on the whitelist by finding all the whitelisted barcodes that are within two differences (Hamming distance <= 2) of the observed sequence, and then scoring them based on the abundance of that barcode in the read data and quality value of the incorrect bases. As another example, an observed barcode that is not present in the whitelist can be corrected to a whitelist barcode if it has > 90% probability of being the real barcode based.

### *Alignment*

The workflow 200 can comprise, at step 215, aligning the read sequences (also referred to as the "reads") to a feature reference file, which specifies all antibodies used and their antibody barcode sequences. For example, the antibody barcode sequences are part of antibody oligonucleotide conjugates. Each of the conjugates comprises an antibody conjugated to a nucleotide sequence that comprises a barcode sequence, a capture sequence, a unique molecular identifier sequence, or a combination thereof. The barcode sequence in the conjugate serves as a reference for alignment of reads.

In the alignment step of the various embodiments herein, a reference-based analysis is performed by aligning a read sequence (also referred to as the "read") to a reference sequence such as an antibody barcode sequence to decide which antibody that read sequence aligns or belongs. All reads that contain the same antibody barcode sequence are reported in the molecule information file.

Various embodiments herein can be configured to correct for sequencing errors in the UMI sequences, before UMI counting. Reads can be placed into groups that share the same barcode, UMI, and gene annotation. If two groups of reads have the same barcode and gene, but their UMIs differ by a single base (i.e., are Hamming distance 1 apart), then one of the UMIs was likely introduced by a substitution error in sequencing. In this case, the UMI of the less-supported read group is corrected to the UMI with higher support.

After grouping the reads by barcode, UMI (possibly corrected), and gene annotation, if two or more groups of reads have the same barcode and UMI, but different gene annotations, the gene annotation with the most supporting reads is kept for UMI counting, and the other read groups can be discarded. In case of a tie for maximal read support, all read groups can be discarded, as the gene cannot be confidently assigned.

After these two filtering steps, each observed barcode, UMI, gene combination is recorded as a UMI count in an unfiltered feature-barcode matrix, which contains every barcode from fixed list of known-good barcode sequences. This includes background and cell-associated barcodes. The number of reads supporting each counted UMI is also recorded in the molecule info file.

### *Unique Molecule Processing*

At step 225, in accordance with various embodiments, to better identify certain subpopulations of cells, a unique molecule processing step can be performed prior to cell calling. The unique molecule processing can include a high content (e.g., antibody content) capture step.

In the high content capture step, the processing can include using a cutoff based on total UMI counts of each barcode to identify cells. This step thus can identify the primary mode of high antibody content cells. This step can include, for example, receiving an expected number of recovered cells, $N$. With $m$ representing the 99th percentile of the top $N$ barcodes by total UMI counts, all barcodes whose total UMI counts exceed $m/10$ can be called as cells in the first pass.

### *Cell Calling*

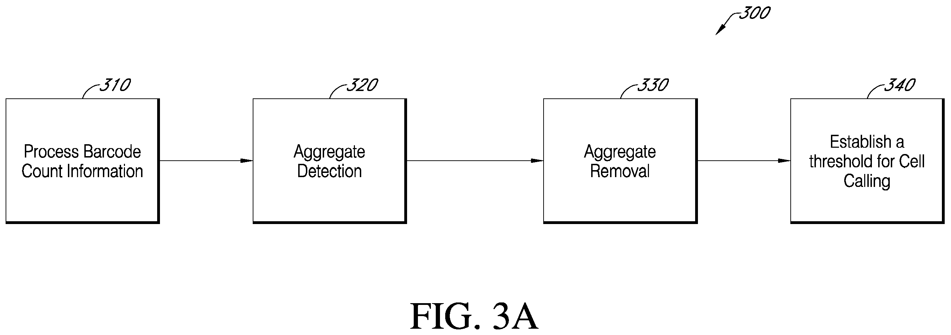

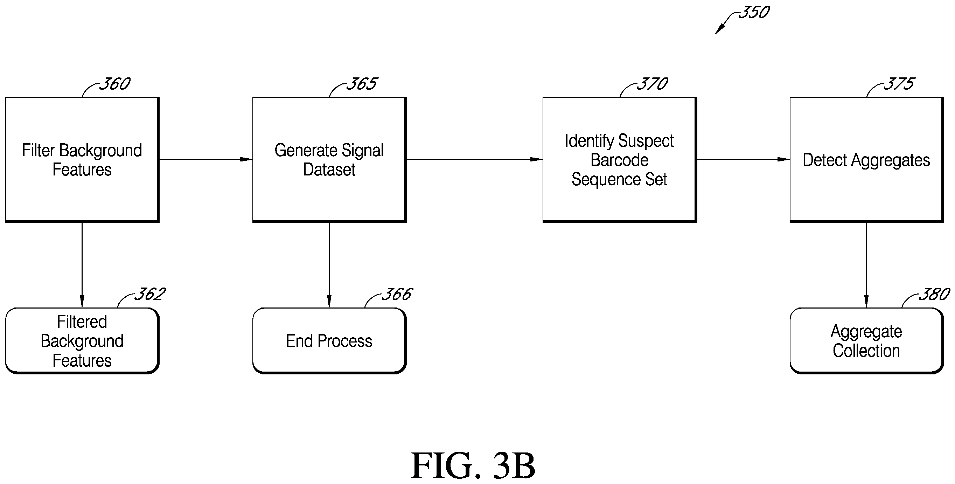

The workflow 200 can comprise, at step 230, a cell calling analysis that includes associating a subset of barcodes observed in the library to the cells loaded from the sample. Identification of these cell barcodes can allow one to then analyze the variation and quantification in data at a single cell resolution. The subset of barcodes can be identified by aggregate detection and removal as discussed in an exemplary workflows FIG. 3a and 3b. The subset of barcodes are likely to be affected by protein aggregates and are identified as aggregate barcodes and can be removed from the first dataset (e.g., starting dataset) to obtain a second dataset of sequence reads for call calling. Cell calling may be used to identify a subset of cells from the second dataset of sequence reads, wherein a cell qualifies a part of the subset of cells if the cell is identified by a barcode in at least one of the second dataset of sequence reads. The subset of cells may be used for further analysis, for example, identifying cell features of the subset of cells.

The process may further include correction of gel bead artifacts, such as gel bead multiples (where a cell shares more than one barcoded gel bead) and barcode multiplets (which occurs when a cell associated gel bead has more than one barcode). In some embodiments, the steps associated with cell calling and correction of gel bead artifacts are utilized together for performing the necessary analysis as part of the various embodiments herein.

In accordance with various embodiments, the record of mapped high-quality fragments that passed all the filters of the various embodiments disclosed in the steps above and were indicated as a fragment in the fragment file (e.g., the fragments.tsv file), are recorded. With the peaks determined in the peak calling step disclosed herein, the number of fragments that overlap any peak regions, for each barcode, can be utilized to separate the signal from noise, i.e., to separate barcodes associated with cells from non-cell barcodes. It is to be understood that such method of separation of signal from noise works better in practice as compared to naively using the number of fragments per barcode.

Various methods, in accordance with various embodiments herein, can be employed to for cell calling. In various embodiments, the cell calling can be performed in two steps. In the first step of cell calling of the various embodiments herein, the barcodes that have fraction of fragments overlapping called peaks lower than the fraction of genome in peaks are identified. When this first step is employed in the cell calling process of the various embodiments herein, the peaks are padded by 2000 bp on both sides to account for the fragment length for this calculation.

### Feature-Barcode Matrix

The workflow 200 can comprise, at step 235, generating a feature-barcode matrix that summarizes that molecular counts (or unique molecular identifier counts) and antibody counts per each cell. The feature-barcode matrix can include only detected cellular barcodes. The generation of the feature-barcode matrix can involve compiling the valid non-filtered UMI counts (e.g., output from the 'Unique Molecule Processing" step discussed herein) from each cell-associated barcode (e.g., output from the "Cell Calling step discussed above) together into the final output count matrix, which can then be used for downstream analysis steps.

### Secondary Analysis

The workflow 200 can comprise, at step 240, various dimensionality reduction, clustering and t-SNE projection tools. Dimensionality reduction tools of the various embodiments herein are utilized to reduce the number of random variables under consideration by obtaining a set of principal variables. In accordance with various embodiments herein, clustering tools can be utilized to assign objects of the various embodiments herein to homogeneous groups (called clusters) while ensuring that objects in different groups are not similar. T-SNE projection tools of the various embodiments herein can include an algorithm for visualization of the data of the various embodiments herein. In accordance with various embodiments, systems and methods within the disclosure can further include dimensionality reduction, clustering and t-SNE projection tools. In some embodiments, the analysis associated with dimensionality reduction, clustering, and t-SNE projection for visualization are utilized together for performing the necessary analysis as part of the various embodiments herein. Various analysis tools for dimensionality reduction such as Principal Component Analysis (PCA), Latent Semantic Analysis (LSA), and Probabilistic Latent Semantic Analysis (PLSA), clustering, and t-SNE projection for visualization that allow one to group and compare a population of cells with another, detail related to which are provided below.

Biological discovery is often aided by visualization tools that allow one to group and compare a population of cells with another. To enable such discovery, various visualization methods within the various embodiments herein, e.g., visualization methods within the Cell Ranger™ analysis pipeline, can be employed. In various embodiments, such visualization methods can include clustering and T-distributed Stochastic Neighbor Embedding (t-SNE) projection tools.

The systems and methods within the disclosure are directed to analyze cell features. As the data is sparse at single cell resolution, dimensionality reduction in accordance with various embodiments herein can be performed to cast the data into a lower dimensional space.

Various systems and methods of various embodiments herein, e.g., systems and methods of the Cell Ranger™ analysis, can be utilized to support dimensionality reduction. Various methods, such as Principal Component Analysis (PCA), Latent Semantic Analysis (LSA), and Probabilistic Latent Semantic Analysis (PLSA), can support dimensionality reduction in accordance with various embodiments herein. In various embodiments, before clustering the cells, LSA is run on the normalized filtered peak-barcode matrix to reduce the number of feature (peak) dimensions. This produces a projection of each cell onto the first N components (default N=15). Thus, various embodiments, the adopted default method of dimensionality reduction is LSA. In other embodiments within the disclosure, users may alternatively choose PCA or PLSA to perform dimensionality reduction in the pipeline. Accordingly, users can specify which dimensionality reduction method to use by providing a dimensionality reduction parameter. In various embodiments, the dimensionality reduction parameter can be "--dim-reduce=<Method>", which can be specified to the various embodiments herein. In accordance with various embodiments herein, each dimensionality reduction method within the disclosure can have an associated data normalization technique that is used prior to the dimensionality reduction step.

In accordance with various embodiments herein, a collection of clustering methods within the disclosure can be employed to accept the dimensionality reduced data. In various embodiments, an optimized implementation of the Barnes Hut TSNE algorithm can be employed to project the dimensionality reduced data into 2-D t-SNE space. In accordance with various embodiments herein, the number of dimensions can be fixed to 15. In various embodiments within the disclosure, it has been observed that fixing the number of dimensions 15 can sufficiently separate clusters visually and in a biologically meaningful way when tested on peripheral blood mononuclear cells (PBMCs). It is understood that other number of dimensions can be fixed in accordance with various embodiments herein. In various embodiments within the disclosure, the number of dimensions (i.e., dimensions of the matrix) can be fixed at a number less than the number of cell-barcodes and the number of peaks. In various embodiments, the number of dimensions can be at least 15. In various embodiments, the number of dimensions can be at least 5, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, or at least 100. It can be understood that higher numbers of dimensions can be computationally costly. Accordingly, computational costs can be determinative of the number of dimensions used. More detail regarding the various methods dimensionality reduction methods described above is provided below.

PCA

When PCA is the dimensionality reduction method of the various embodiments herein, the data is normalized to median cut site counts per barcode and log-transformed. In various embodiments, a fast, scalable and memory efficient implementation of IRLBA (Augmented, Implicitly Restarted Lanczos Bidiagonalization Algorithm) can be used that allows in-place centering and feature scaling and produces the transformed matrix along with the principal components (PC) and singular values encoding the variance explained by each PC. When PCA is the default dimensionality reduction method of the various embodiments herein, k-means clustering can be used. In various embodiments, k-means clustering produces 2 to 10 clusters for visualization and analysis. In another embodiment within the disclosure, a k-nearest neighbors graph-based clustering method is also provided via community detection using a modularity optimization algorithm. In various embodiments, the modularity optimization algorithm is louvain modularity optimization algorithm. The transformed matrix of the various embodiments herein, can be operated on by the t-SNE algorithm with default parameters (e.g., tsne_input_pcs, tsne_perplexity, tsne_theta, tsne_max_dims, tsne_max_iter, tsne_stop_lying_iter, and tsne_mom_switch_iter) and can provide 2-D coordinates for each barcode for visualization with various embodiments herein.

Below is a summary of default parameters of t-SNE algorithm in accordance with various embodiments.

| Parameter | Type | Default Value | Recommended Range | Description |
|---|---|---|---|---|
| tsne_input_pcs | int | null | Cannot be set higher than the num_comps parameter, which is N principal components for LSA/PCA/PLSA. | Subset to top N principal components for TSNE. |
| tsne_perplexity | int | 30 | 30-50 | TSNE perplexity parameter. |
| tsne_theta | float | 0.5 | Between 0 and 1. | TSNE theta parameter. |
| tsne_max_dims | int | 2 | 2 or 3. | Maximum number of TSNE output dimensions. |
| tsne_max_iter | int | 1000 | 1000-10000 | Number of total TSNE iterations. |
| tsne_stop_lying_iter | int | 250 | Cannot be set higher than tsne_max_iter. | Iteration at which TSNE learning rate is reduced. |

(continued)

| Parameter | Type | Default Value | Recommended Range | Description |
|---|---|---|---|---|
| tsne_mom_switch_iter | int | 250 | Cannot be set higher than tsne_max_iter. | Iteration at which TSNE momentum is reduced. |

LSA

In accordance with various embodiments herein, the data can be normalized via the inverse-document frequency (idf) transformer, where each peak count can be scaled by the log of the ratio of the number of barcodes in the matrix and the number of barcodes where the peak has a non-zero count. This normalization can provide greater weight to counts in peaks that occur in fewer barcodes. In some embodiments within the disclosure, singular value decomposition (SVD) can be performed on this normalized matrix using IRLBA without scaling or centering, to produce the transformed matrix in lower dimensional space, as well as the components and the singular values signifying the importance of each component. In some embodiments within the disclosure, prior to clustering, normalization to depth can be performed by scaling each barcode data point to unit L2-norm in the lower dimensional space. It has been observed that the combination of these normalization techniques obviates the need to remove the first component in accordance with various embodiments herein. When LSA is the dimensionality reduction method of the various embodiments herein, a spherical k-means clustering can be provided that produces 2 to 10 clusters for downstream analysis. It has been observed that spherical k-means can perform better than plain k-means, by identifying clusters via k-means on L2-normalized data that can live on the spherical manifold. Accordingly, spherical k-means can be suitable to cluster large-scale datasets from aggregation runs, as described in detail below. In another embodiment within the disclosure, and similar to PCA, a graph-based clustering can be provided and visualized via t-SNE. However, similar to spherical k-means clustering, the data can be normalized to unit norm before performing graph-based clustering and t-SNE projection.

PLSA

PLSA is a special type of non-negative matrix factorization, with roots in Natural Language Processing. When PLSA is the dimensionality reduction method of the various embodiments herein, the KL-divergence between the empirically determined probability of observing a peak in a barcode and the lower rank approximation to it is minimized, via an Expectation-Maximization algorithm. In various embodiments, the data is not normalized prior to dimensionality reduction via PLSA. Similar to LSA and PCA, a transformed matrix, component vectors, and a set of values explaining the importance of each component can be produced in accordance with various embodiments herein. PLSA can offer natural interpretation of the components and the transformed matrix of the various embodiments herein. In accordance with various embodiments herein, each component can be interpreted as a hidden topic and the transformed matrix can simply be the probability of observing a barcode from a given topic (i.e., Prob(barcode|topic)). In accordance with various embodiments herein, the component vectors can be the probability of observing a peak from a given topic (i.e., (Prob(peak|topic)) and the counterpart to singular values of LSA/PCA can simply be the probability of each topic (i.e., (Prob(topic)) observed in the data of various embodiments herein. Similar to LSA, the transformed matrix for PLSA can be normalized to unit L2-norm. In various embodiments, spherical k-means clustering can be performed to produce 2 to 10 clusters. In another embodiment within the disclosure, graph-based clustering can also be performed. The transformed matrix of the various embodiments herein, can then be visualized via t-SNE. It is understood that while PLSA offers great advantages in interpretability of the lower dimensional space, it is appreciably slower than both PCA and LSA and does not scale well beyond 20 components on large datasets. To ameliorate this to some extent, in various embodiments, the in-house implementation of PLSA can be multithreaded (4 threads on compute cluster) and written and compiled in C++. To ensure a reasonable run time, in various embodiments, the algorithm can be capped at 3000 iterations in the event it does not converge first. It is understood that other number of dimensions can be fixed in accordance with various embodiments herein.

It is understood that various clustering methods including, but not limited to, K-Means clustering, affinity propagation, mean-shift, spectral clustering, Ward hierarchical clustering, agglomerative clustering, DBSCAN, OPTICS, Gaussian mixture models, Birch clustering, and k-medoids clustering, and visualization approaches can be utilized in accordance with various embodiments herein. It is understood that each clustering method may have various tradeoffs. Accordingly, in various embodiments, selection of a clustering method can be made based on whether the clusters make biological sense with known, well-studied sample types (e.g., PBMCs), i.e., whether the clusters generated using a particular clustering method make sense with validation on known biology. Below is a non-limiting summary of dimensionality reduction techniques and associated clustering and visualization approaches in accordance with various embodiments herein.

| Dimensionality Reduction | Clustering | Visualization |
|---|---|---|
| PCA | K-means, graph-clustering | TSNE |
| LSA | Spherical k-means, graph-clustering | TSNE |
| PLSA | Spherical k-means, graph-clustering | TSNE |

### *Differential Expression Analysis*

In accordance with various embodiments, the workflow 200 can comprise, at step 245, a differential expression analysis that performs differential analysis to identify features specific to each cluster, Cell Ranger tests, for each feature and each cluster, whether the in-cluster mean differs from the out-of-cluster mean.

To find differentially expressed features between groups of cells, systems and methods disclosed herein can use the quick and simple method sSeq (Yu, D., Huber, W. & Vitek, O. "Shrinkage estimation of dispersion in Negative Binomial models for RNA-seq experiments with small sample size" Bioinformatics 29, 1275-1282 (2013)), which employs a negative binomial exact test. When the counts become large, systems and methods disclosed herein switches to the fast asymptotic beta test used in edgeR (Robinson, M. D. & Smyth, G. K. "Small-sample estimation of negative binomial dispersion, with applications to SAGE data" Biostatistics 9, 321-332 (2007)). For each cluster, the algorithm is run on that cluster versus all other cells, yielding a list of features that are differentially expressed in that cluster relative to the rest of the sample.

The implementation of the systems and methods disclosed herein may incorporate, or even differs from that in the sSeq paper where the authors recommend using DESeq's geometric mean-based definition of library size. The systems and methods disclosed herein can, instead, compute relative library size as the total UMI counts for each cell divided by the median UMI counts per cell. As with sSeq, normalization is implicit in that the per-cell library-size parameter is incorporated as a factor in the exact-test probability calculations.

### *Aggregate Detection and Removal*

FIG. 3a provides an exemplary workflow 300 for aggregate detection and removal, in accordance with various embodiments. In step 310, received barcode count information is processed.

Steps 320 and 330 can be conducted to ensure cell calling quality to reduce sequencing and library preparation costs. Proteins can aggregate together and form a large population of aggregates, which can phenotypically result in certain cells binding an exceedingly high amount of proteins aggregates. Such protein aggregates would severely deteriorate the quality of cell calling. Steps 320 and 330 are designed to remove barcodes that are affected by protein aggregates before cell calling.

Step 320 can be conducted to detect aggregate barcodes. At step 320, a subset of barcodes in the first dataset can be identified as aggregates. In the analysis, each sequence read has a barcode sequence. The sequence reads are grouped into bins, wherein each bin comprises sequence reads that share a common barcode sequence. The step 320 identifies a subset of barcode sequences from the bins as aggregates, wherein a bin is placed into the subset of barcodes when a percentage of correction events in the bin meets a pre-set criterion for such a percentage.

In various embodiments, a correction event occurs, or a sequence read is identified as being corrected if (a) the sequence read differs in a set number of nucleotides (e.g., one or more) from one or more other sequence reads in the bin, and (b) the one or more other sequence reads are removed from the total sequence reads. There can be two or more sequence reads that have share the same barcode sequence as being in the same bin, and that differ in a set number of nucleotides (e.g., only or at most one, two, three, four, five, six, seven, eight, nine, ten nucleotides or any numbers or ranges derived therefrom).

Correction events of each barcode will be tracked. In some embodiments, when sequence reads in a barcode bin have had a correction event or are being corrected, only one sequence read will remain for future analysis and will be identified as being corrected for a calculation of a correction rate, and all other sequence reads that differ in a set number of nucleotide in the same bin will be removed from future analysis. In exemplary embodiments, a correction event occurs, or a sequence read is identified as being corrected, if the sequence read differs in one nucleotide (e.g., are Hamming distance 1 apart) from one or more other sequence reads in the bin. In additional and alternative embodiments, a correction event occurs, or a sequence read is identified as being corrected, if (a) the sequence read differs in one nucleotide (e.g., are Hamming distance 1 apart) from one or more other sequence reads in the bin, and (b) the one or more other sequence reads are removed from the total sequence reads. If the reads have the same barcode, but they differ in one single base, then one of the sequence reads were likely introduced by a substitution error in sequencing.

In accordance with embodiments, the step 320 can alternatively identify a subset of barcode sequences from the bins as aggregates, wherein a bin is placed into the subset of barcodes when (a) a percentage of correction events in the bin meets a pre-set criterion for such a percentage and (b) a number of total sequence reads in the bin exceeds or equals to a pre-set threshold of total sequence reads.

In accordance with various embodiments, a method for removing aggregates from a dataset is provided. The method can comprise receiving, by one or more processors, a first dataset comprising a plurality of sequence reads. The method can comprise grouping, by the one or more processors, the plurality of sequence reads into bins, wherein each bin comprises sequence reads that share a common barcode sequence. The method can comprise identifying, by the one or more processors, a subset of barcode sequences from the bins, wherein a bin is placed into the subset of barcode sequences when a percentage of correction events in the bin meets a pre-set criterion for such a percentage, wherein a correction event occurs if a sequence read differs in one or more nucleotides from one or more other sequence reads in the bin. The method can further comprise removing, by the one or more processors, the subset of barcode sequences from the first dataset to obtain a second dataset of sequence reads, and generating, by the one or more processors, an output comprising the second dataset of sequence reads.

In accordance with various embodiments, the method can further comprise identifying a first subset of cells from the second dataset of sequence reads, wherein a cell qualifies as part of the first subset of cells if the cell is identified by a barcode in at least one of the second dataset of sequence reads.

In accordance with various embodiments, a bin can be into the subset of barcode sequences when at least 50% of total sequence reads in the bin have had a correction event.

In accordance with various embodiments, the method can further comprise ranking barcodes in the second dataset based on molecular counts of each barcode in the second dataset and determining a threshold value of molecular counts for selecting barcodes using a pre-set percentile of ranked barcodes in the second dataset, wherein any barcodes in the second dataset having a molecular count above the threshold value are selected to obtain a third dataset of sequence reads for cell calling. The method can further comprise identifying a second subset of cells from the third dataset of sequence reads, wherein a cell qualifies as part of the second subset of cells if the cell is identified by a barcode in at least one of the third dataset of sequence reads. The pre-set percentile of ranked barcodes can be the 1st percentile barcode of ranked barcodes. The threshold value can be 10 percent of a molecular count of the 1st percentile barcode of ranked barcodes.

In accordance with various embodiments, the bin can be placed into the subset of barcode sequences when (a) a percentage of correction events in the bin meets a pre-set criterion for such a percentage; and (b) a number of total sequence reads in the bin exceeds or equals to a pre-set threshold of total sequence reads. The pre-set threshold of the number of total sequence reads can be 10,000 total sequence reads.

In accordance with various embodiments, the first dataset can be obtained from an antibody capture library.

In accordance with various embodiments, at step 320, barcodes can be identified as aggregates based on number of UMIs, rather than correction events as discussed herein. Identifying aggregates based on number of UMIs can be done in tandem with aggregate identification as a function of correction events. Alternatively, identifying aggregates based on number of UMIs can be done instead of aggregate identification as a function of correction events.

The UMI number approach can proceed under the assumption (though not required) that normally when customers run large enough antibody panels (large as discussed in more detail below), most antibodies in that panel will stain different cell types or, in other words, most cell types will specifically bind to only a few antibodies from the panel. Thus, one may not expect to see a lot of antibodies of different types on any barcode - and if such a barcode is detected, it may more likely be the result of protein aggregation, since that often may be the most reasonable explanation as to why a partition (e.g., a GEM) would contain high counts of completely unrelated antibodies.

In accordance with various embodiments, FIG. 3b provides an exemplary workflow 350 for aggregate detection as a function of UMI count, in accordance with various embodiments. At step 360, background features (e.g., antibodies, and can be referred to as antibodies herein) in the dataset are filtered out at step 362. Background antibodies can be filtered out by considering antibodies that exceed a given UMI threshold. Such considered antibodies can be referred to as signal antibodies (see step 365). For example, the UMI threshold can be such that only antibodies with higher than about 1000 UMIs are considered, and thus are labeled as signal antibodies and advance in the workflow. The UMI threshold value, however, can vary depending on experiment type and can be greater or lesser than 1000.

Moreover, at step 365, if the number of signal antibodies identified is less than a given signal feature threshold (e.g., signal antibody threshold), then workflow 350 can move to an end process 366. In accordance with various embodiments, the given signal antibody threshold can be set, for example, to five or less signal antibodies. Of course, the threshold can be user set to even higher values than five, depending on the experiment. It should be noted that while steps 360 and 365 are illustrated as independent steps, these steps can be also run as one individual step, whereby, at step 360 (or 365 for that matter), the workflow includes removing, by the one or more processors, from the dataset, features having UMI counts that that do not exceed a given UMI threshold, to produce a signal dataset including those features exceeding the given UMI threshold.

As discussed herein, this workflow can proceed under the assumption (though not required), usually true for large enough panels, that users usually target, for example, diverse cell types. However, in those circumstances where a user only runs, for example, two, three or four antibodies, the user might be trying to stain similar cell types. Furthermore, in those circumstances where, for example, two to three antibodies are detected on a barcode, it may be more difficult to differentiate between genuine protein aggregation or cell multiplets. As such, setting the signal antibody threshold to require high counts of, for example, at least five antibodies before declaring a barcode as an aggregate can assist in improving the accuracy of the aggregate detection method. However, it should be noted that the value of five antibodies is an example of a signal threshold value but, as stated above, can be set to values less than five, such as, for example, three and four antibodies, depending on the experiment.

In step 370, a set of suspect barcodes are identified, in accordance with various embodiments. Suspect barcodes can be selected as a function of total antibody UMI counts. For example, the top 25 barcodes by total antibody UMI counts for a given dataset can be selected as "suspect" barcodes for further analysis. The top 25 barcodes by total antibody UMI count can be referred to as a barcode threshold. The barcode threshold, however, can be set above or below 25 as desired by user.

In step 375, each suspect barcode is analyzed to determine if the barcode is an aggregate. This analysis can be conducted using, for example, a two-part test. In part one, a determination is made of how many antibodies should be detected to capture the suspect barcode for further aggregate analysis. A key parameter for this determination is the total number of signal antibodies (discussed and determined above) in the dataset. Using that total signal antibody number, a detected feature threshold (e.g., detected antibody threshold) can be calculated for a barcode.

In accordance with various embodiments, the detected feature threshold can be calculated using, for example, a linear model that takes in the total number of signal features (e.g., antibodies) and calculates the associated detected feature threshold. For example, if a customer stained with five antibodies, using the linear model, a detected feature threshold of five antibodies would be set on the candidate barcodes. In another example, if 10 antibodies are used, a detected feature threshold of nine antibodies would be set on the candidate barcodes. In another example, if 20 antibodies are used, a detected feature threshold of 14 antibodies would be set on the candidate barcodes. If 25 antibodies are used, a detected feature threshold of 15 antibodies would be set on the candidate barcodes.

An example of a linear model can be explained as follows. For example, given the total number of antibodies used in an experiment, a linear model used can include coefficients that satisfy the following: 100% of a five-antibody panel (i.e., for five total antibodies, the threshold is the detection of all five antibodies) and 60% of a 25-antibody panel (i.e., for 25 total antibodies, the threshold is the detection of 15 antibodies). This percentage (or fraction) can then decrease monotonically in between these two data points (five and 25 antibody panels). Moreover, the linear model can be set whereby the percentage (detected feature threshold) can be set to about 60% for about 25 antibodies. The associated equations can be as follows:

$$m * 5 + b = 1.0$$

$$m * 25 + b = 0.6,$$

where m = -0.02 and b = 1.1

Applying this linear model across all panels between five and 25 antibodies results in the following conversion table:

| Total Antibodies | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|
| Detected Antibody Threshold | 5 | 6 | 7 | 8 | 8 | 9 | 10 | 11 | 11 | 12 | 12 | 13 | 13 | 14 | 14 | 14 | 15 | 15 | 15 | 15 | 15 |

Next, the second part of the two-part test determines whether the determined antibodies (which meet the detected antibody threshold value, e.g., from table above) are in high enough counts. For example, and as discussed above regarding suspect barcode identification, the same empirical threshold can be used, namely, that the UMI counts of any antibody on a given barcode can be considered suspicious if the UMI count is within the top 25 barcodes for the given antibody. Effectively, the 25th highest UMI count for each antibody across all barcodes can be considered an aggregate UMI threshold, a value which, if exceeded for each of the determined antibodies up to the detected antibody threshold for a given barcode in a given experiment, results in the suspicious barcode being labeled as an aggregate. The aggregate UMI threshold, however, can be set above or below the 25th highest UMI count as desired by user.

The collection of aggregates can then be gathered, at step 380, for downstream processing and removal as will be discussed below.

An example application of this two-part test is as follows. For a 17-antibody panel, a first step is to look at all antibodies with higher than 1000 UMIs. There are 10 such antibodies. Focusing on the top 25 barcodes by their TOTAL antibody counts, each barcode is examined. Using, for example, the table above, for 10 total antibodies, if any 9 antibodies have UMI counts that are in the top 25 UMI counts for each antibody across all barcodes, then the given barcode is called an aggregate.

An example application of the overall workflow illustrated in FIG. 3b and discussed above, is as follows. In this example, an experiment is conducted where five antibodies were used - CD3, CD19, CD4, CD8a, and CD14. For each of these antibodies, the 25th highest UMI count across all barcodes is, respectively for each of the five antibodies, 1000, 5000, 2000, 3000, and 500. Next, the top 25 barcodes by total counts is examined as suspect barcodes for aggregate labeling. If the first barcode has counts [1251, 5346, 2700, 3900, 12000], this would be considered an aggregate as each of the five antibodies crossed its required threshold (of 1000, 5000, 2000, 3000 and 500 respectively). If the second barcode has counts [4089, 7891, 2800, 3781, 490], this barcode would not be considered an aggregate. Even though the first four antibodies were present in high enough counts, the fifth antibody was not (490 less than 500). As stated in the above table, for a five-antibody experiment, the detected antibody threshold is also five antibodies. Thus, per the table, all five would have to exceed their 25th highest counts on any barcode to declare an aggregate.

The workflow described above and exemplified by FIG. 3b can operate independently to identify aggregates for removal at step 330 described below. Alternatively, the workflow described above and exemplified by FIG. 3b can operate together with the workflow described above and exemplified by FIG. 3a, to identify aggregates for removal at step 330 described below. It is understood that (a) either or both workflows could detect zero aggregate barcodes, (b) some of the detected duplicates can be duplicates that were detected by both workflows, or (c) all barcodes detected between workflows can be unique to each individual detection workflow.

Step 330 can be conducted to remove barcodes that are identified as aggregates. After tracking correction events and identifying aggregate barcodes, a subset of barcode sequences that are identified as aggregates and all their molecule counts are removed from future analysis. An output comprising a new dataset of sequences reads with aggregate barcodes removed can be generated for future analysis. All the reads are still maintained in a molecule info file, regardless whether they are from aggregates or not.

At step 340, an optional filter step may be performed to establish a threshold for cell calling. For example, a cutoff based on total UMI counts of each barcode can be used to identify cells. In some examples, this step identifies the primary mode of high antibody content cells. Examples of cells/samples can include, for example, PBMCs (Peripheral Blood Mononuclear Cells), cell lines, primary cell samples, dissociated tumor cells, primary neurons, fibroblasts, cardiomyocytes, cultured neurons, embryonic cell samples, samples comprising cells from multiple species, immune cells Isolated from tumor cells or patient samples, FACS-sorted cells, and isolated nuclei samples (which are not technically complete cells).

In step 340, a threshold such as a count cut off value is determined. To determine the cutoff, the processing can include using a cutoff based on total UMI counts of each barcode to identify cells. This step can include, for example, receiving an expected number of recovered cells, $N$. With $m$ representing the top 1st percentile of the top $N$ sorted barcodes by total UMI counts, the count value of $m/10$ determines the high cutoff. The pre-set percentile is a parameter that can be varied. For example, the pre-set percentile can range from about 0.5% to about 20%, from about 1% to about 10%, any iterative ranges in between, about 1%, and so on. Because of the possible interrelated nature of pre-set percentile and fold change parameter (e.g., the m/10 value for the denominator - by default equal to 10), an adjustment to the accompanying fold-change parameter can take place. For example, for an N value of 3000 barcodes, the top 1st percentile of the barcodes would be the 30th ranked barcode by UMI count. If that UMI count of the 30th ranked barcode was 500 (i.e., $m$=500), the $m/10$ high count cutoff would be a UMI count of 50, with any barcodes having a UMI count exceeding that value being considered cells in this cell calling analysis. The denominator is a parameter that can be varied. For example, the denominator can range from about 2 to about 50, about 3 to about 20, any iterative ranges in between, about 10, and so on.

Steps 320 (Aggregate Detection), 330 (Aggregate Removal), and 340 (Establish a Threshold for Cell Calling) can be part of the Unique Molecular Processing step 225 in FIG. 2 or can be performed between the Unique Molecular Processing step 225 and the Cell Calling step 230, or can be part of the Cell Calling step 230.

In various embodiments, methods are provided for identifying a dataset for cell calling. The methods can be implemented via computer software or hardware. The methods can also be implemented on a computing device/system that can include a combination of engines for identifying a subset of cells from a dataset for cell feature analysis. In various embodiments, the computing device/system can be communicatively connected to one or more of a data source, sample analyzer (e.g., a genomic sequence analyzer), and display device via a direct connection or through an internet connection.

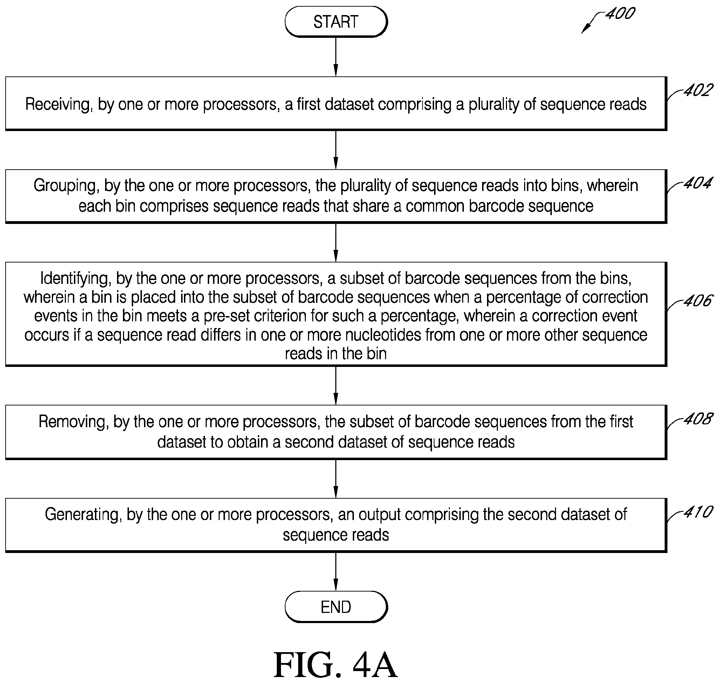

Referring now to FIG. 4a, a flowchart illustrating a non-limiting example method 400 for identifying a dataset for cell calling is disclosed, in accordance with various embodiments. The method can comprise, at step 402, receiving a first dataset comprising a plurality of sequence reads. The first dataset can be obtained from an antibody capture library or any library using labeling agents to study cells or cell features.

The methoFd can comprise, at step 404, grouping the plurality of sequence reads into bins, wherein each bin comprises reads that share a common barcode sequence. This step may provide groups of bins with sequence reads that have varying degrees of correction events. In the same bin, some sequence reads may be identified as being corrected or have had a correction event, and some other sequence reads may be identified as not being corrected or have not had any correction events, and they may be used to calculate a correction rate by dividing a number of correction events by a total number of sequence read in the bin.

The method can comprise, at step 406, identifying a subset of barcode sequences from the grouped bins, wherein a bin is placed into the subset of barcode sequences when a percentage of correction events in the bin meets a pre-set criterion for such a percentage. For example, a bin can be placed into the subset of barcode sequences when at least 50%, 60%, 70%, 80% or any intermediate range or percentage of total sequence reads in the bin have been had a correction event.

In additional and alternative embodiments, the bin can be placed into the subset of barcode sequences as aggregates when (a) a percentage of correction events in the bin meets a pre-set criterion for such a percentage; and (b) a number of total sequence reads in the bin exceeds or equals to a pre-set threshold of total sequence reads. For example, the pre-set threshold of total sequence reads is 10, 000 sequence reads. The pre-set threshold of total sequence reads can be 5,000 to 100,000 or any values or ranges derivable therefrom.

In one particular example, the bin can be placed into the subset of barcode sequences if two criteria are met: (a) at least 50% of total sequence reads in the bin have had a correction event; (b) the bin has at least 10,000 total sequence reads.

In various embodiments, a sequence read is identified as having had a correction event if the sequence read differs in one nucleotide from one or more other sequence reads in the bin. In additional and alternative embodiments, a sequence read is identified as having had a correction event if (a) the sequence read differs in one nucleotide from one or more other sequence reads in the bin and (b) the one or more other sequence reads are removed from the total sequence reads so the sequence read is the only one remaining for correction rate analysis. When several sequence reads were identified as only differing in one nucleotide from one sequence read in the bin, only one sequence read stays and will counted toward a correction rate, and all other sequence reads were corrected. This correction process can be repeated or performed at the same time to result in many correction events.

In additional and alternative embodiments, a correction rate can be calculated by dividing a raw count of corrected sequence reads by a number of total sequence reads. When a group of several sequence reads are identified in the bin as only differing in one nucleotide from at least another one read in the group, all the sequence reads in the group can be counted as having had been corrected and can all count toward a correction rate.

Correction of sequence reads and tracking of such correction can be performed before step 404, after step 404, be part of step 404, before step 406, or be part of step 406.

The method can comprise, at step 408, removing sequence reads having the subset of barcode sequences from the first dataset to obtain a second dataset of sequence reads for cell calling.

The method can comprise, at step 410, generate an output comprising the second dataset of sequence reads that have the aggregate barcodes removed. The method can further comprise identifying a first subset of cells from the second dataset of sequence reads, wherein a cell qualifies as part of the first subset of cells if the cell is identified by a barcode in at least one of the second dataset of sequence reads. The method can further comprise measuring cell features in the first subset of cells.

In additional and alternative embodiments, the method can further comprise ranking barcodes in the second dataset based on molecular counts of each barcode in the second dataset and determining a threshold value of molecular counts for selecting barcodes using a pre-set percentile of ranked barcodes in the second dataset, wherein any barcodes in the second dataset having a molecular count above the threshold value are selected to obtain a third dataset of sequence reads for cell calling. For example, the pre-set percentile of ranked barcodes can be the 1st percentile barcode of the barcodes in the second dataset. The method can further comprise identifying a second subset of cells from the third dataset of sequence reads, wherein a cell qualifies as part of the second subset of cells if the cell is identified by a barcode in at least one of the third dataset of sequence reads. For example, the threshold value is 10 percent of a molecular count of the 1st percentile barcode.

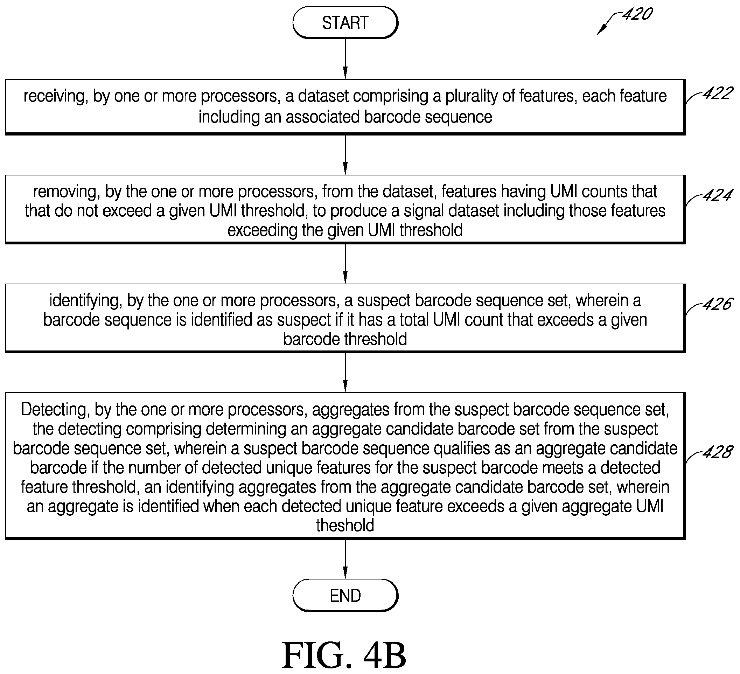

In accordance with various embodiments, FIG. 4b illustrates a non-limiting example method 420 for identifying a dataset for cell calling.

The method can include, at step 422, receiving a dataset comprising a plurality of features, each feature including an associated barcode sequence

The method can include, at step 424, removing, from the dataset, features having UMI counts that that do not exceed a given UMI threshold, to produce a signal dataset including those features exceeding the given UMI threshold.

The method can include, at step 426, identifying a suspect barcode sequence set, wherein a barcode sequence is identified as suspect if it has a total UMI count that exceeds a given barcode threshold.

The method can include, at step 428, detecting aggregates from the suspect barcode sequence set. The detecting can include determining an aggregate candidate barcode set from the suspect barcode sequence set, wherein a suspect barcode sequence qualifies as an aggregate candidate barcode if the number of detected unique features for the suspect barcode meets a detected feature threshold. The detecting can further include identifying aggregates from the aggregate candidate barcode set, wherein an aggregate is identified when each detected unique feature exceeds a given aggregate UMI threshold.

In accordance with various embodiments, each feature can be associated with an antibody.

In accordance with various embodiments, the detected feature threshold can be a function of the number of features in the signal dataset. For example, for a signal dataset with 25 features, the detected feature threshold can be 15 features. For example, for a signal dataset with five features, the detected feature threshold can be five features. For example, the detected feature threshold can be between about 60% and 100% of the number of features in the signal dataset. For example, the detected feature threshold can be set to about 60% for a signal dataset over 25 features.

In accordance with various embodiments, the UMI threshold can be about 1000 UMIs.

In accordance with various embodiments, the barcode threshold can be the top 25 barcodes across the dataset by total UMI count.

In accordance with various embodiments, the aggregate UMI threshold can be the 25th highest barcode.

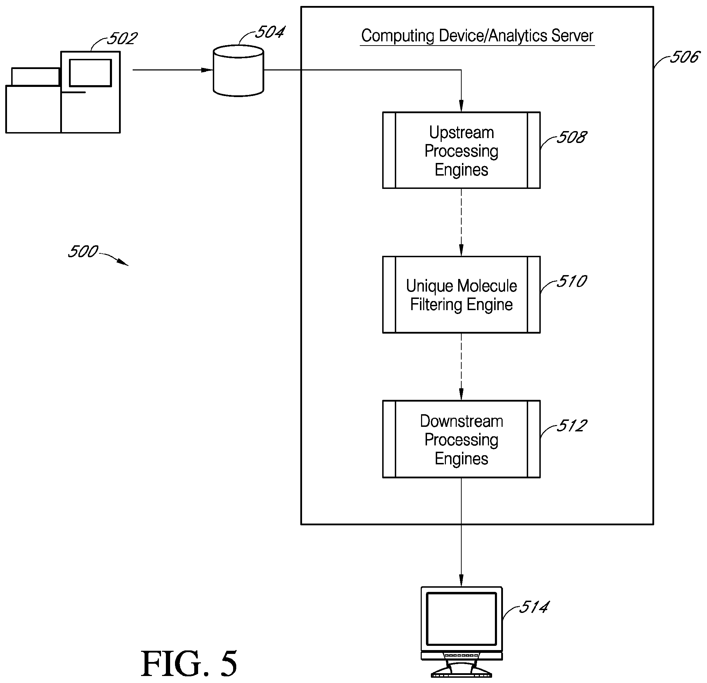

In accordance with various embodiments, FIG. 5 illustrates a non-limiting example system for detecting aggregates from a dataset, in accordance with various embodiments. The system 500 includes a sequence analyzer 502, a data storage unit 504, a computing device/analytics server 506, and a client terminal/display 514.

The genomic sequence analyzer 502 can be communicatively connected to the data storage unit 504 by way of a serial bus (if both form an integrated instrument platform) or by way of a network connection (if both are distributed/separate devices). The genomic sequence analyzer 502 can be configured to process, analyze and generate one or more datasets from a sample, such as an antibody capture library of the various embodiments herein. Each sequence read in the library includes a barcode and unique identifier sequence (i.e., UMI). In various embodiments, the genomic sequence analyzer 502 can be a next-generation sequencing platform or sequencer such as the Illumina® sequencer, MiSeq™, NextSeq™ 500/550 (High Output), HiSeq 2500™ (Rapid Run), HiSeq™ 3000/4000, and NovaSeq.

In various embodiments, the generated genomic sequence datasets can then be stored in the data storage unit 504 for subsequent processing. In various embodiments, one or more raw genomic sequence datasets can also be stored in the data storage unit 504 prior to processing and analyzing. Accordingly, in various embodiments, the data storage unit 504 can be configured to store one or more genomic sequence datasets, e.g., the genomic sequence datasets of the various embodiments herein can include a plurality of fragment sequence reads with their associated barcodes and unique identifier sequences that also can comprise feature data, wherein each feature can include the associated barcode sequence and/or an additional barcode sequence. In various embodiments, the processed and analyzed genomic sequence datasets comprising feature data with associated barcodes can be fed to the computing device/analytics server 506 in real-time for further downstream analysis.

In various embodiments, the data storage unit 504 is communicatively connected to the computing device/analytics server 506. In various embodiments, the data storage unit 504 and the computing device/analytics server 506 can be part of an integrated apparatus. In various embodiments, the data storage unit 504 can be hosted by a different device than the computing device/analytics server 506. In various embodiments, the data storage unit 504 and the computing device/analytics server 506 can be part of a distributed network system. In various embodiments, the computing device/analytics server 506 can be communicatively connected to the data storage unit 504 via a network connection that can be either a "hardwired" physical network connection (e.g., Internet, LAN, WAN, VPN, etc.) or a wireless network connection (e.g., Wi-Fi, WLAN, etc.). In various embodiments, the computing device/analytics server 506 can be a workstation, mainframe computer, distributed computing node (part of a "cloud computing" or distributed networking system), personal computer, mobile device, etc.

In various embodiments, the computing device/analytics sever 506 is configured to host one or more upstream data processing engines 508, a Unique Molecule Filtering Engine 510, and one or more downstream data processing engines 512.

Examples of upstream data processing engines 508 can include, but are not limited to: alignment engine, cell barcode processing engine (for correcting sequencing barcode sequencing errors), etc.

The Unique Molecule Filtering Engine 510 can be configured to receive one or more datasets comprising a plurality of features (feature dataset), each feature including an associated barcode sequence that can be stored in the data storage unit 504. The feature datasets can be comprised of a plurality of fragment sequence reads including feature data (generated from the sequencing of a fragment library, for example, an antibody capture library), each with an associated barcode sequence and a unique identifier sequence (i.e., UMI). In various embodiments, the Unique Molecule Filtering Engine 510 can be configured to receive processed and analyzed sequence datasets from the genomic sequence analyzer 502 in real-time.

In various embodiments, the Unique Molecule Filtering Engine 510 can be configured to remove, from the dataset, features having UMI counts that that do not exceed a given UMI threshold

In various embodiments, the Unique Molecule Filtering Engine 510 can be configured to generate a signal dataset including those features exceeding the given UMI threshold.

In various embodiments, the Unique Molecule Filtering Engine 510 can be configured to identify a suspect barcode sequence set, wherein a barcode sequence is identified as suspect if it has a total UMI count that exceeds a given barcode threshold.

In various embodiments, the Unique Molecule Filtering Engine 510 can be configured to detect aggregates from the suspect barcode sequence set, the detecting comprising determining an aggregate candidate barcode set from the suspect barcode sequence set, wherein a suspect barcode sequence qualifies as an aggregate candidate barcode if the number of detected unique features for the suspect barcode meets a detected feature threshold, and identifying aggregates from the aggregate candidate barcode set, wherein an aggregate is identified when each detected unique feature exceeds a given aggregate UMI threshold.

In various embodiments, each feature can be associated with an antibody.

In various embodiments, the detected feature threshold can be a function of the number of features in the signal dataset. In various embodiments, the detected feature threshold can be 15 features. In various embodiments, the detected feature threshold can be five features. In various embodiments, the detected feature threshold can be between about 60% and 100% of the number of features in the signal dataset.

In various embodiments, the UMI threshold can be about 1000 UMIs. In various embodiments, the barcode threshold can be the top 25 barcodes across the dataset by total UMI count. In various embodiments, the aggregate UMI threshold can be the $25^{th}$ highest barcode.

The detected aggregates from the suspect barcode sequence can then be further processed by one or more downstream data processing engines 512 for the purposes of cell feature analysis. Examples of downstream data processing engines 512 can include, but are not limited to: cell calling engine (for grouping fragment sequence reads as being from a unique cell), feature barcode matrix engine (for creating a feature barcode matrix), differential analysis engine (for identifying features specific to each cell cluster), etc. In certain embodiments, parts of the unique molecular filtering engine 510 can be combined with or be part of the cell calling engine in the downstream processing engine 512.

After the downstream processing has been performed and an output of the results can be displayed as a result or summary on a display or client terminal 514 that is communicatively connected to the computing device/analytics server 506. In various embodiments, the display or client terminal 514 can be a thin client computing device. In various embodiments, the display or client terminal 514 can be a personal computing device having a web browser (e.g., INTERNET EXPLORER™, FIREFOX™, SAFARI™, etc.) that can be used to visualize the operation of the genomic sequence analyzer 502, data store 504, upstream data processing engines 508, unique molecule filtering engine 510, and the downstream data processing engines 512.

It should be appreciated that the various engines can be combined or collapsed into a single engine, component or module, depending on the requirements of the particular application or system architecture. In various embodiments engines 508/510/512 can comprise additional engines or components as needed by the particular application or system architecture.

## COMPUTER IMPLEMENTED SYSTEM

In various embodiments, the methods for removing aggregates from a dataset can be implemented via computer software or hardware. That is, as depicted in FIG. 5, the methods disclosed herein can be implemented on a computing device 506 that includes upstream processing engines 508, a unique molecule filtering engine 510 and downstream processing engines 512. In various embodiments, the computing device 506 can be communicatively connected to a data store 504 and a client terminal/display device 514 via a direct connection or through an internet connection.

It should be appreciated that the various engines depicted in FIG. 5 can be combined or collapsed into a single engine, component or module, depending on the requirements of the particular application or system architecture. Moreover, in various embodiments, the upstream processing engines 508, unique molecule filtering engine 510 and downstream processing engines 512 can comprise additional engines or components as needed by the particular application or system architecture.

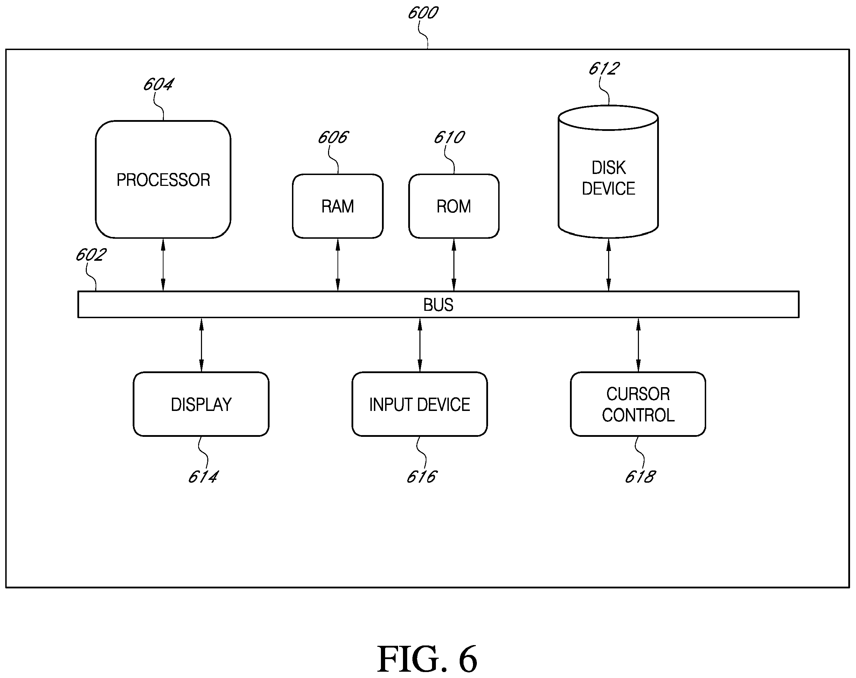

FIG. 6 is a block diagram illustrating a computer system 600 upon which embodiments of the present teachings may be implemented. In various embodiments of the present teachings, computer system 600 can include a bus 602 or other communication mechanism for communicating information and a processor 604 coupled with bus 602 for processing information. In various embodiments, computer system 600 can also include a memory, which can be a random-access memory (RAM) 606 or other dynamic storage device, coupled to bus 602 for determining instructions to be executed by processor 604. Memory can also be used for storing temporary variables or other intermediate information during execution of instructions to be executed by processor 604. In various embodiments, computer system 600 can further include a read only memory (ROM) 608 or other static storage device coupled to bus 602 for storing static information and instructions for processor 604. A storage device 610, such as a magnetic disk or optical disk, can be provided and coupled to bus 602 for storing information and instructions.

In various embodiments, computer system 600 can be coupled via bus 602 to a display 614, such as a cathode ray tube (CRT) or liquid crystal display (LCD), for displaying information to a computer user. An input device 616, including alphanumeric and other keys, can be coupled to bus 602 for communication of information and command selections to processor 604. Another type of user input device is a cursor control 618, such as a mouse, a trackball or cursor direction keys for communicating direction information and command selections to processor 604 and for controlling cursor movement on display 614. This input device 616 typically has two degrees of freedom in two axes, a first axis (i.e., x) and a second axis (i.e., y), that allows the device to specify positions in a plane. However, it should be understood that input devices 616 allowing for 3-dimensional (x, y and z) cursor movement are also contemplated herein.

Consistent with certain implementations of the present teachings, results can be provided by computer system 600 in response to processor 604 executing one or more sequences of one or more instructions contained in memory 606. Such instructions can be read into memory 606 from another computer-readable medium or computer-readable storage medium, such as storage device 612. Execution of the sequences of instructions contained in memory 606 can cause processor 604 to perform the processes described herein. Alternatively, hard-wired circuitry can be used in place of or in combination with software instructions to implement the present teachings. Thus, implementations of the present teachings are not limited to any specific combination of hardware circuitry and software.

The term "computer-readable medium" (e.g., data store, data storage, etc.) or "computer-readable storage medium" as used herein refers to any media that participates in providing instructions to processor 604 for execution. Such a medium can take many forms, including but not limited to, non-volatile media, volatile media, and transmission media. Examples of non-volatile media can include, but are not limited to, dynamic memory, such as memory 606. Examples of transmission media can include, but are not limited to, coaxial cables, copper wire, and fiber optics, including the wires that comprise bus 602.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, another memory chip or cartridge, or any other tangible medium from which a computer can read.

In addition to computer-readable medium, instructions or data can be provided as signals on transmission media included in a communications apparatus or system to provide sequences of one or more instructions to processor 604 of computer system 600 for execution. For example, a communication apparatus may include a transceiver having signals indicative of instructions and data. The instructions and data are configured to cause one or more processors to implement the functions outlined in the disclosure herein. Representative examples of data communications transmission connections can include, but are not limited to, telephone modem connections, wide area networks (WAN), local area networks (LAN), infrared data connections, NFC connections, etc.

It should be appreciated that the methodologies described herein, flow charts, diagrams and accompanying disclosure can be implemented using computer system 600 as a standalone device or on a distributed network or shared computer processing resources such as a cloud computing network.

The methodologies described herein may be implemented by various means depending upon the application. For example, these methodologies may be implemented in hardware, firmware, software, or any combination thereof. For a hardware implementation, the processing unit may be implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, electronic devices, other electronic units designed to perform the functions described herein, or a combination thereof.

In various embodiments, the methods of the present teachings may be implemented as firmware and/or a software program and applications written in conventional programming languages such as C, C++, Python, etc. If implemented as firmware and/or software, the embodiments described herein can be implemented on a non-transitory computer-readable medium in which a program is stored for causing a computer to perform the methods described above. It should be understood that the various engines described herein can be provided on a computer system, such as computer system 600, whereby processor 604 would execute the analyses and determinations provided by these engines, subject to instructions provided by any one of, or a combination of, memory components 606/610/612 and user input provided via input device 616.

While the present teachings are described in conjunction with various embodiments, it is not intended that the present teachings be limited to such embodiments.

In describing the various embodiments, the specification may have presented a method and/or process as a particular sequence of steps. However, to the extent that the method or process does not rely on the particular order of steps set forth herein, the method or process should not be limited to the particular sequence of steps described, and one skilled in the art can readily appreciate that the sequences may be varied and still remain within the scope of the various embodiments.

**Claims**

1. A method for detecting aggregates from a dataset, the method comprising:

   receiving (422), by one or more processors, a dataset comprising a plurality of features, each feature including an associated barcode sequence;
   removing (424), by the one or more processors, from the dataset, features having UMI counts that that do not exceed a given UMI threshold, to produce a signal dataset including those features exceeding the given UMI threshold;
   identifying (426), by the one or more processors, a suspect barcode sequence set, wherein a barcode sequence is identified as suspect if it has a total UMI count that exceeds a given barcode threshold;
   detecting (428), by the one or more processors, aggregates from the suspect barcode sequence set, the detecting comprising:

      determining, by the one or more processors, an aggregate candidate barcode set from the suspect barcode sequence set, wherein a suspect barcode sequence qualifies as an aggregate candidate barcode if the number of detected unique features for the suspect barcode meets a detected feature threshold, wherein the detected feature threshold is a function of the number of features in the signal dataset, and
      identifying, by the one or more processors, aggregates from the aggregate candidate barcode set, wherein an aggregate is identified when each detected unique feature exceeds a given aggregate UMI threshold.

2. The method of claim 1, wherein each feature is associated with an antibody.

3. The method of claim 1 or claim 2, wherein for a signal dataset with 25 features, the detected feature threshold is 15 features.

4. The method of any preceding claim, wherein for a signal dataset with five features, the detected feature threshold is five features.

5. The method of any preceding claim, wherein the detected feature threshold is between about 60% and 100% of the number of features in the signal dataset.

6. The method according to any one of claims 1-5, wherein the UMI threshold is about 1000 UMIs.

7. The method of claim 1, wherein the barcode threshold is the top 25 barcodes across the dataset by total UMI count.

8. The method according to any one of claims 1-5, wherein the aggregate UMI threshold is the 25th highest barcode.

9. A computer-readable medium storing computer instructions that, when executed by a computer, cause the computer to perform the method of any preceding claim.

10. A system (500) for detecting aggregates from a dataset, the system comprising:

    a data store (504) configured to store a dataset comprising a plurality of features, each feature including an associated barcode sequence; and
    a computing device (506) communicatively connected to the data store, comprising a unique molecule filtering engine configured to perform the method of any of claims 1 to 8.

**Patentansprüche**

1. Verfahren zum Erkennen von Aggregaten aus einem Datensatz, wobei das Verfahren umfasst:

   Empfangen (422), durch einen oder mehrere Prozessor(en), eines Datensatzes, der eine Vielzahl von Merkmalen umfasst, wobei jedes Merkmal eine Sequenz assoziierter Strichcodes einschließt;

Entfernen (424), durch den einen oder die mehreren Prozessor(en), von Merkmalen mit UMI-Zählungen, die eine gegebene UMI-Schwelle nicht überschreiten, aus dem Datensatz, um einen Signaldatensatz zu erzeugen, der diejenigen Merkmale, die die gegebene UMI-Schwelle überschreiten, einschließt;

Identifizieren (426), durch den einen oder die mehreren Prozessor(en), eines Satzes verdächtiger Strichcodesequenzen, wobei eine Strichcodesequenz als verdächtig identifiziert wird, wenn sie eine Gesamt-UMI-Zählung aufweist, die eine gegebene Strichcodeschwelle überschreitet;

Erkennen (428), durch den einen oder die mehreren Prozessor(en), von Aggregaten aus dem Satz verdächtiger Strichcodesequenzen, wobei das Erkennen umfasst:

Bestimmen, durch den einen oder die mehreren Prozessor(en), eines Satzes möglicher aggregierter Strichcodes aus dem Satz verdächtiger Strichcodesequenzen, wobei eine verdächtige Strichcodesequenz als ein möglicher aggregierter Strichcode in Frage kommt, wenn die Anzahl erkannter eindeutiger Merkmale für den verdächtigen Strichcode eine Schwelle erkannter Merkmale erreicht, wobei die Schwelle erkannter Merkmale von der Anzahl von Merkmalen im Signaldatensatz abhängig ist, und

Identifizieren, durch den einen oder die mehreren Prozessor(en), von Aggregaten aus dem Satz möglicher aggregierter Strichcodes, wobei ein Aggregat identifiziert wird, wenn jedes erkannte eindeutige Merkmal eine gegebene aggregierte UMI-Schwelle überschreitet.

2. Verfahren nach Anspruch 1, wobei jedes Merkmal mit einem Antikörper assoziiert ist.

3. Verfahren nach Anspruch 1 oder Anspruch 2, wobei für einen Signaldatensatz mit 25 Merkmalen die Schwelle erkannter Merkmale 15 Merkmale beträgt.

4. Verfahren nach einem der vorhergehenden Ansprüche, wobei für einen Signaldatensatz mit fünf Merkmalen die Schwelle erkannter Merkmale fünf Merkmale beträgt.

5. Verfahren nach einem der vorhergehenden Ansprüche, wobei die Schwelle erkannter Merkmale zwischen etwa 60 % und 100 % der Anzahl von Merkmalen im Signaldatensatz beträgt.

6. Verfahren nach einem der Ansprüche 1 bis 5, wobei die UMI-Schwelle etwa 1000 UMIs beträgt.

7. Verfahren nach Anspruch 1, wobei der Strichcodeschwellenwert die oberen 25 Strichcodes über den Datensatz nach Gesamt-UMI-Zählung ist.

8. Verfahren nach einem der Ansprüche 1 bis 5, wobei die Gesamt-UMI-Schwelle der 25.-höchste Strichcode ist.

9. Computerlesbares Medium, das Computeranweisungen speichert, die, wenn sie durch einen Computer ausgeführt werden, den Computer zum Durchführen des Verfahrens nach einem der vorhergehenden Ansprüche veranlassen.

10. System (500) zum Erkennen von Aggregaten aus einem Datensatz, wobei das System umfasst:

einen Datenspeicher (504), der dazu ausgelegt ist, einen Datensatz zu speichern, der eine Vielzahl von Merkmalen umfasst, wobei jedes Merkmal eine Sequenz assoziierter Strichcodes einschließt; und

eine Rechenvorrichtung (506), die kommunikativ mit dem Datenspeicher verbunden ist und eine spezifische Molekülfilterungs-Engine umfasst, die dazu ausgelegt ist, das Verfahren nach einem der Ansprüche 1 bis 8 durchzuführen.

## Revendications

1. Procédé de détection d'agrégats à partir d'un ensemble de données, le procédé comprenant :

la réception (422), par un ou plusieurs processeurs, d'un ensemble de données comprenant une pluralité d'entités, chaque entité comprenant une séquence de code-barres associée ;

le fait de retirer (424) de l'ensemble de données, par les un ou plusieurs processeurs, des entités ayant des comptages UMI qui ne dépassent pas un seuil UMI donné, afin de produire un ensemble de données filtré comportant les entités dépassant le seuil UMI donné ;

l'identification (426), par les un ou plusieurs processeurs, d'un ensemble de séquences de codes-barres suspectes, où une séquence de code-barres est identifiée comme suspecte si elle a un nombre total d'UMI qui dépasse un seuil de code-barres donné ;

la détection (428), par les un ou plusieurs processeurs, d'agrégats à partir de l'ensemble de séquences de codes-barres suspectes, la détection comprenant :

la détermination, par les un ou plusieurs processeurs, d'un ensemble de codes-barres candidat agrégé à partir de l'ensemble de séquences de codes-barres suspectes, une séquence de code-barres suspecte étant qualifiée de code-barres candidat agrégé si le nombre d'entités uniques détectées pour le code-barres suspect atteint un seuil d'entités détectées, le seuil d'entités détectées étant fonction du nombre d'entités dans l'ensemble de données filtré, et

l'identification, par les un ou plusieurs processeurs, des agrégats de l'ensemble de codes-barres candidat agrégé, un agrégat étant identifié lorsque chaque entité unique détectée dépasse un seuil UMI agrégé donné.

2. Procédé selon la revendication 1, dans lequel chaque entité est associée à un anticorps.

3. Procédé selon la revendication 1 ou la revendication 2, dans lequel, pour un ensemble de données filtré avec 25 entités, le seuil d'entités détectées est 15 entités.

4. Procédé selon l'une quelconque des revendications précédentes, dans lequel, pour un ensemble de données filtré avec cinq entités, le seuil d'entités détectées est cinq entités.

5. Procédé selon l'une quelconque des revendications précédentes, dans lequel le seuil d'entités détectées est compris entre environ 60 % et 100 % du nombre d'entités dans l'ensemble de données filtré.

6. Procédé selon l'une quelconque des revendications 1 à 5, dans lequel le seuil UMI est d'environ 1000 UMI.

7. Procédé selon la revendication 1, dans lequel le seuil de code-barres correspond à l'ensemble des 25 codes-barres présentant les nombres totaux d'UMI les plus élevés dans l'ensemble de données.

8. Procédé selon l'une quelconque des revendications 1 à 5, dans lequel le seuil UMI agrégé correspond au code-barres présentant le 25$^{ième}$ code-barres le plus élevé.

9. Support lisible par ordinateur stockant des instructions informatiques qui, lorsqu'elles sont exécutées par un ordinateur, amènent l'ordinateur à exécuter le procédé selon l'une quelconque des revendications précédentes.

10. Système (500) pour détecter des agrégats à partir d'un ensemble de données, le système comprenant :

une mémoire de données (504) configurée pour stocker un ensemble de données comprenant une pluralité d'entités, chaque entité comprenant une séquence de code-barres associée ; et

un dispositif informatique (506) connecté de manière communicative à la mémoire de données, et comprenant un moteur de filtrage de molécules uniques configuré pour exécuter le procédé selon l'une quelconque des revendications 1 à 8.

FIG. 1

225 Unique Molecule Processing

230 Cell Calling

200

235 Feature-Barcode Matrix

215 Alignment

240 Secondary Analysis (Dimensionality Reduction, Clustering, t-SNE Projection, etc.)

210 Barcode Processing

245 Differential Expression Analysis

FIG. 2

*300*

| *310* | *320* | *330* | *340* |
|---|---|---|---|
| Process Barcode Count Information | Aggregate Detection | Aggregate Removal | Establish a threshold for Cell Calling |

FIG. 3A

$\overset{\displaystyle 350}{\diagup}$

```
        ┌─────────────┐      ┌─────────────┐      ┌─────────────┐      ┌─────────────┐
        │ 360         │      │ 365         │      │ 370         │      │ 375         │
        │   Filter    │      │  Generate   │      │  Identify   │      │             │
        │ Background  │ ───▶ │   Signal    │ ───▶ │   Suspect   │ ───▶ │   Detect    │
        │  Features   │      │   Dataset   │      │   Barcode   │      │ Aggregates  │
        │             │      │             │      │Sequence Set │      │             │
        └──────┬──────┘      └──────┬──────┘      └─────────────┘      └──────┬──────┘
               │                    │                                        │
               ▼                    ▼                                        ▼
        ┌─────────────┐      ┌─────────────┐                          ┌─────────────┐
        │ 362         │      │ 366         │                          │ 380         │
        │  Filtered   │      │             │                          │  Aggregate  │
        │ Background  │      │ End Process │                          │ Collection  │
        │  Features   │      │             │                          │             │
        └─────────────┘      └─────────────┘                          └─────────────┘
```

EP 4 186 060 B1

# FIG. 3B

EP 4 186 060 B1

( START ) — 400

Receiving, by one or more processors, a first dataset comprising a plurality of sequence reads — 402

Grouping, by the one or more processors, the plurality of sequence reads into bins, wherein each bin comprises sequence reads that share a common barcode sequence — 404

Identifying, by the one or more processors, a subset of barcode sequences from the bins, wherein a bin is placed into the subset of barcode sequences when a percentage of correction events in the bin meets a pre-set criterion for such a percentage, wherein a correction event occurs if a sequence read differs in one or more nucleotides from one or more other sequence reads in the bin — 406

Removing, by the one or more processors, the subset of barcode sequences from the first dataset to obtain a second dataset of sequence reads — 408

Generating, by the one or more processors, an output comprising the second dataset of sequence reads — 410

( END )

FIG. 4A

START — 420

receiving, by one or more processors, a dataset comprising a plurality of features, each feature including an associated barcode sequence — 422

removing, by the one or more processors, from the dataset, features having UMI counts that that do not exceed a given UMI threshold, to produce a signal dataset including those features exceeding the given UMI threshold — 424

identifying, by the one or more processors, a suspect barcode sequence set, wherein a barcode sequence is identified as suspect if it has a total UMI count that exceeds a given barcode threshold — 426

Detecting, by the one or more processors, aggregates from the suspect barcode sequence set, the detecting comprising determining an aggregate candidate barcode set from the suspect barcode sequence set, wherein a suspect barcode sequence qualifies as an aggregate candidate barcode if the number of detected unique features for the suspect barcode meets a detected feature threshold, an identifying aggregates from the aggregate candidate barcode set, wherein an aggregate is identified when each detected unique feature exceeds a given aggregate UMI theshold — 428

END

FIG. 4B

Computing Device/Analytics Server

506

508 Upstream Processing Engines

510 Unique Molecule Filtering Engine

512 Downstream Processing Engines

514

504

502

500

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019236599 A2
- US 10550429 B
- US 20190177800 A
- US 20190367969 A
- WO 2006084132 A
- US 87319010
- US 87313210
- US 316383
- US 14316398
- US 14316416
- US 14316431
- US 14316447
- US 14316463 B
- US 10343166 B
- US 10583440 B
- US 20180179590 A1
- US 20190367969 A1
- US 20200002763 A1
- US 20200002764 A1
- WO 2019040637 A
- US 20190323088 A

### Non-patent literature cited in the description

- **SAMBROOK et al.** Molecular Cloning: A Laboratory Manual. Cold Spring Harbor Laboratory Press, 2000
- **YU, D.** ; **HUBER, W.** ; **VITEK, O.** Shrinkage estimation of dispersion in Negative Binomial models for RNA-seq experiments with small sample size. *Bioinformatics*, 2013, vol. 29, 1275-1282
- **ROBINSON, M. D.** ; **SMYTH, G. K.** Small-sample estimation of negative binomial dispersion, with applications to SAGE data. *Biostatistics*, 2007, vol. 9, 321-332